# EUROPEAN PATENT APPLICATION

(11) **EP 4 480 580 A1**
(43) Date of publication of application: **25.12.2024**
(21) Application number: 23181319.7
(22) Date of filing: 23.06.2023
(51) Int. Cl.: B01L 3/00, C12M 3/06, C12M 1/00

(54) **A FLUIDIC DEVICE**

(71) Applicant: Koninklijke Philips N.V., 5656 AG Eindhoven (NL)
(72) Inventor: BECKERS, Lucas Johannes Anna Maria, 5656 AG Eindhoven (NL); LIPSCH, Job, 5656 AG Eindhoven (NL); SANDERS, Rene, 5656AG Eindhoven (NL); BERBEN, Edward Theodorus Maria, 5656 AG Eindhoven (NL); VULDERS, Roland Cornelis Martinus, 5656 AG Eindhoven (NL); VALSTER, Susanne Maaike, 5656 AG Eindhoven (NL)
(74) Representative: Philips Intellectual Property & Standards

(57) **Abstract**

A fluidic device comprising a monolithic substrate. The substrate defines a first chamber, a second chamber and a fluid channel connecting the two chambers. The first chamber supports a perforated membrane that divides the first chamber into a first sub-chamber and a second sub-chamber. The second chamber is configured for supporting a cell culture. The substrate is configured such that fluid introduced to the second chamber flows to the first sub-chamber of the first chamber, before passing through the perforated membrane to the second sub-chamber. Thus, fluid introduced to the first sub-chamber via the fluid channel can only reach the second sub-chamber via the perforated membrane.

## Description

### FIELD OF THE INVENTION

The invention relates to fluidic devices for culturing and investigating cells.

### BACKGROUND OF THE INVENTION

In-vitro testing of mammalian cells or tissue is an important technique. For example, biopsied mammalian cells or tissue may be subjected to such testing to determine anomalies or disease in such mammalian material or may be exposed to drugs, e.g., experimental drugs, to monitor the response of the diseased mammalian material. The latter approach is frequently used in oncological procedures. This can provide important insights in how a disease in an individual can be effectively treated without having to expose the individual itself to a range of potentially effective drugs. After all, such exposure can be undesirable for several reasons. One reason being the occurrence of adverse side effects based on drug toxicity. Another reason being uncertainty on the efficacy of experimental drugs for existing diseases for which no established satisfactory drug treatment is yet available.

A common approach to such in-vitro testing is to immobilize the mammalian material in a fluidic device, which is sometimes also referred to as an "organ-on-chip". In such an approach, the mammalian material is typically immobilized on a membrane separating two sub-chambers of the fluidic device. One sub-chamber is used to contain the mammalian material and the other sub-chamber is used to expose the mammalian material to a chemical compound or composition of interest such as a drug treatment, e.g. to test the efficacy and/or toxicity of the drug as previously explained.

An example of the microfabrication of a human organ-on-chip based on thin film techniques is disclosed by Dongeun Huh et al. in Nature Protocols, Vol. 8, No. 11, 2013, pages 2135-2157.

It would be advantageous to provide fluidic devices that can be used for other forms of drug-effect assessment. In particular, improved devices for cancer drug testing are desired.

### SUMMARY OF THE INVENTION

The invention is defined by the claims.

According to examples in accordance with an aspect of the disclosure, there is provided a fluidic device as claimed in claim 1.

The device comprises an integrally formed substrate. The integrally formed substrate comprises a first chamber for housing a perforated membrane and a second chamber for culturing cells or tissue. The fluidic device comprises the perforated membrane within the substrate such that it divides the first chamber into a first sub-chamber and a second sub-chamber. The fluidic device further comprises a first fluid channel connecting the first sub-chamber to the second chamber such that fluid can be made to flow from the second chamber to the first sub-chamber via the first fluid channel. The perforated membrane comprises a plurality of pores having a diameter of 10 µm or less.

The fluidic device allows the creation of a fluid flow (e.g. by an appropriate pumping system) from the second chamber to the first sub-chamber to transport cells, e.g. emitted from the cell culture, if any, to the first sub-chamber to be retained or aggregated therein by the perforated membrane due to their small size pores that allow fluid passage but not target cell passage. Such flow could mimic for example a blood flow.

The device is therefore suitable for investigation of cell emission or release behavior of a cancerous cell culture or tissue under various conditions including variations to nourishment and/or exposure to drugs as with chemotherapy. Released and separately captured cells, if any, may be analyzed and/or treated independently from the cell cultures and tissues. The proposed fluidic device thus provides an integrated device for the study of metastasizing cell cultures or tissues and treatment thereof. All this may be conveniently done within the same fluidic device because of the high level of integration of the fluidic device and substrate. This has many advantages as described herein after, one of which is reduced dead-space and loss of cells as well as reduced amounts of fluids needed. In addition, the high level of integration in combination with chamber, channel and perforated membrane design allows for improved methods of manufacture with respect to for example reproducibility and cost. In some cases, when the membrane is made of certain transparent and/or non-autofluorescing elastomer materials, optical investigation using for example microscopy is enabled.

In some examples one or more of the fluid channel and the perforated membrane is part of the integrally formed substrate. This increases the level of integration even more. Other, optional chambers and channels may also be part of the integrally formed substrate.

In some examples the first chamber is no less than 0.5 mm wide; and the second chamber is no less than 1 mm wide. In some examples each of the pores of the perforated membrane has a width or diameter in the range of 2 µm to 10 µm, preferably in the range of 2 to 9 µm. The width or diameter may be chosen based on type of cells to be retained or captured by the perforated membrane.

In some examples the fluidic device further comprises a supporting member for supporting the cell culture, the supporting member arranged in the substrate to divide the second chamber into a third sub-chamber and a second sub-chamber such that the third sub-chamber is connected to the first sub-chamber via the first fluid channel. The third sub-chamber, as part of the second chamber, is now the chamber for the cell culturing.

In some examples the perforated membrane is a first perforated membrane and the supporting member comprises a second perforated membrane comprising a plurality of second pores each having a diameter of 10 µm or less.

In a fluidic device having the first, second, third and fourth sub-chambers as well as the first and second perforated membrane, therapy for treatment (e.g. any drugs) can be provided to the first sub-chamber and third sub-chamber via the respective perforated membranes while nourishment for cell culturing for example may be in dependently provided not through any perforated membranes. In addition, therapy for treatment may be provided to the cell culture and to the emitted cells independently form each other.

In some examples the supporting member or if not present the second chamber comprises a plurality of separate wells, each well for culturing a separate one of a plurality of cell cultures, each well having an opening facing the third sub-chamber. Each well opening may have a diameter in the range of 200 µm to 1000 µm. Each well can have a portion of the second perforated membrane with pores having a width of 10 µm or less. The wells may be sized and shaped to each contain substantially one spheroid or organoid. Since the location of the wells is known, the location of their contents is also well known. This facilitates faster optical analysis and may cause less drift of the contents of the wells under fluid flow.

In some examples the supporting member comprises a slit or other mechanism for holding a biopsy.

In preferred examples the fluidic device further comprises one or more optional channels. The optional channels comprising:
- a first input fluid channel 350 connected to the second chamber and a first inlet for introducing fluid to the second chamber ; and
- a first output fluid channel connected to the second sub-chamber and a first outlet for removing fluid from the second sub-chamber; and
- a second input fluid channel connected to the fourth sub-chamber and a second inlet for removing fluid from the fourth sub-chamber; and
- a second output fluid channel connected to the first sub-chamber and a second outlet for removing fluid from the first sub-chamber.

Preferably all of the optional channels are incorporated and one or more and preferably all of these are part of the integrated substrate. Any of the channels may be connected to a fluid inlet or outlet as defined herein.

In some examples a fluidic device having a supporting member further comprises a bridging channel connecting the second sub-chamber to the fourth sub-chamber such that fluid can flow between the second and fourth sub-chamber via the bridging channel.

This provides a flow of fluid from fourth sub-chamber to second sub-chamber such that the contents of these chambers may be changed without fluid in them having to pass any perforated membranes.

In some examples the fluidic device comprises a bypass channel connected to the fourth sub-chamber and a fluid outlet. The fluid outlet may be a fluid outlet of the second chamber, but this is not necessary. The bypass channel allows change of content of the fourth chamber independent of content of the second chamber.

In preferred examples the fluidic device of any of the previous claims comprise a plurality of dual chamber units wherein a dual chamber unit is defined by at least the first sub-chamber, the second sub-chamber, the perforated membrane, the second chamber, the first fluid channel and, if present, the supporting member. This provides a compact fluidic device that may be used for parallel experimentation. Such experimentation may for example involve using equipment as known in the art for manipulation and analysis of contents of standard well cell plates.

In some examples the plurality of dual chamber units are arranged such that:
- their first sub-chambers are connected in parallel to a joint second fluid output channel connected to a second outlet;
- their second sub-chambers are connected in series to a joint first output fluid channel between a further inlet and a first outlet.

In some examples the plurality of dual chamber units are arranged such that:
- their third sub-chambers are connected in parallel to a joint first input fluid channel connected to a first inlet; and,
- optionally, when the fluidic device comprises a supporting member as defined in claim 4, their fourth sub-chambers are connected in series to a joint second input fluid channel between a second inlet and a further outlet.

Interconnections allows reduction of fluid inlets and outlets while parallel connected units can be used for simultaneous experiments. The interconnections of fourth sub-channels provide a first flow system that can be independently operated from the interconnections of the second sub-channels. Either of these may be independently operated from the flow system including any series connection of first sub-chamber, first fluid channel and third sub-channel. This allows many complicated metastasis investigation protocols to be performed with the substrate.

In preferred examples the perforated membrane comprises an elastomeric material, preferably a polysiloxane based elastomeric material. Preferably such material is transparent for visible light and/or has reduced or not autofluorescence.

In some examples the fluidic device has a visible light transmittance of 0.8 or more. Preferably such transmission is measured at a chamber location and along a light path including the perforated membrane. This increases an ease for a user of the fluidic device to monitor and/or assess any cells or particles captured by the perforated membrane or elsewhere in the fluidic device.

There is also proposed a method as claimed in claim 16. The method may be a method for testing therapy or drug on cell emission. The method may include optional steps such as exposing the emitted cells to therapy or drugs, where such drug may be different from drugs or therapy to which the cell culture was exposed.

The fluidic device may comprise an output fluid channel fluidly connected to the second sub-chamber to allow the output or extraction of fluid from the second sub-chamber. *This allows waste fluid to be siphoned away from the first chamber, e.g., allowing for fluid to continually flow through to the perforated membrane and avoiding spillage.*

In preferred examples, the first chamber is connected to the second chamber via only one or more channels formed in a single side of the fluidic device. *This technique provides a simple, materially efficient and easy to manufacture technique that provides many, if not all, of the required fluid channels for assessing the effect of metastasis.*

Preferably, only a single fluid channel connects the first chamber to the second chamber. However, this is not essential.

In at least one example, the integrally formed substrate comprises an input fluid channel extending from the second chamber and only fluidly connected to the first chamber via the second chamber; and the input fluid channel comprises an exposed portion for receiving fluid for the second chamber.

Preferably, the integrally formed substrate comprises a first planar surface and the first and second chambers are formed as recesses in the first planar surface.

*The substrate of the fluidic device comprises a substrate formed of a single piece of material which substrate comprises at least one pair of chambers and, preferably at least one fluid channel that connects the pair of chambers together. One of the chambers (the "first chamber") comprises (houses or supports) a perforated membrane allowing passage of fluid. The perforated membrane may be, and preferably is, integrally formed with the substrate (e.g. be formed as part of the substrate), or be a separate element (e.g. be formed separately and inserted into the substrate). The fluidic device is designed such that fluid can flow from one chamber (the second chamber) in which cells or tissue can be cultured to the other chamber (the first chamber) to carry or transport released cells (if any) to the perforated membrane where the fluid can pass the membrane while at least some of the released cells (if any) are retained by the perforated membrane. The device is configured such that fluid flow can be set to mimic a blood flow in organisms, for example by external fluid pump. The fluidic device is thus configured to mimic a blood (like) flow. The cells carried by the fluid are captured by the perforated membrane due to the relatively small size of the pores therein. The captured cells can then be investigated separately from the cell culture or tissue. Such analysis can be done using optical analysis equipment, e.g., by microscope or the like, to analyze or assess their condition, number and*/*or shape.*

*In this way, it is possible to assess cells that have been released or emitted by any cell cultures or tissue provided in the second chamber. The second chamber can therefore be used to perform a treatment on the cell culture(s), such as the application of a certain therapeutic fluid or drug (e.g., a chemotherapy treatment fluid), with the first chamber capturing any released cells to analyze the effect of the treatment performed in the second chamber on metastasis.*

### BRIEF DESCRIPTION OF THE DRAWINGS

For a better understanding of the claimed aspects, and to show more clearly how they may be carried into effect, reference will now be made, by way of example only, to the accompanying schematic drawings not drawn to scale, in which:
Figs. 1A to 1C illustrate examples of a fluidic device, where Figs. 1B and 1C provide a cross-sectional views along cross-sectional plane 109 of the fluidic device shown in Fig. 1A;
Figs. 2A and 2B provide cross-sectional views along a cross-sectional plane 109 of a fluidic device similar to that shown in Fig. 1A but with different chamber and channel configurations.
Figs. 3A to 3C illustrate further examples of fluidic devices;
Fig. 4 illustrates a portion of an example fluidic device having wells;
Figs. 5A to 5D illustrate an example of a fluidic device where Fig. 5A provides a first/upper side view of the fluidic device and Fig. 5B provides a second/lower side view of the fluidic device and Fig. 5C shows how cover plates may be arranged;
Figs. 6A and 6B illustrate an example of a fluidic device having multiple dual chamber units;
Figs. 6C and 6D illustrate normal and transparent mode top views of the same magnified part of the fluidic device of Figs. 6A and 6B;
Figs. 7A and 7B illustrate flow diagrams of methods of use of fluidic devices disclosed herein;
Fig. 8 illustrates a system for testing a cell culture;

### DETAILED DESCRIPTION OF THE EMBODIMENTS

The invention will be described with reference to the Figs.

The detailed description and specific examples, while indicating exemplary embodiments of the devices, systems and methods, are intended for purposes of illustration and are not intended to limit the scope of the claims. These and other features, aspects, and advantages of the devices, systems and methods disclosure herein will become better understood from the following description, appended claims, and accompanying drawings. The same reference numerals are used throughout the Figs. to indicate the same or similar parts.

The disclosure provides a fluidic device, sometimes also referred to as "microfluidic device". The device comprises an integrally formed substrate also sometimes referred to herein as "body", where the substrate comprises a first chamber, a second chamber and, optionally, but preferably a first fluid channel fluidically connecting the first and second chambers.

The substrate is an integrally formed one where "integrally formed" means a substrate formed of one piece of material. Such material preferably is an elastic material such as an optically transparent elastic material. An integrally formed substrate is sometimes also referred to as a "monolithic substrate". Thus, the first and second chambers and, optionally but preferably also the first fluid channel are integrally formed as a part of the substrate. Optionally other channels and features are also formed as part of the integrally formed substrate as will be further exemplified hereinafter.

A "chamber" and "fluid channel" are herein used to refer to spaces or volumes that are completely enclosed or partly enclosed by the substrate. The chambers and fluid channels defined herein are configured to comprise (or capable of comprising) liquids, such as for example watery liquids, and/or, sometimes, gasses such as e.g, oxygen or carbon dioxide.

A "connection" in the context of the current disclosure means any connection that allows fluid passage where fluid preferably comprises water or sometimes gasses such as oxygen or carbon dioxide.

The substrate comprises a perforated membrane that divides the first chamber into a first sub-chamber and a second sub-chamber. The membrane may be integrally formed with the substrate or added as a separately manufactured piece.

The second chamber is for supporting a cell culture. It is designed to contain a cell culture and provide it with nourishment such as to keep the cell culture alive or to cause it to grow proliferate etc. A cell culture may be a piece of tissue such as biopsy, a spheroid or organoid or other.

The second chamber is connected to the first sub-chamber of the first chamber by a first fluid channel.

This first fluid channel is configured such that fluid introduced to the second chamber must flow via the first fluid channel to the first sub-chamber before it can pass through the perforated membrane to the second sub-chamber. The first fluid channel has dimensions large enough to allow passage of at least single cells.

The perforated membrane has one or more, preferably a plurality of pores wide enough to allow fluid flow therethrough and narrow enough to retain cells. This enables the perforated membrane to provide a cell filtering function. Therewith cells emitted from the cell culture in the second chamber may be transported by a fluid flow from the second chamber to the first sub-chamber and be retained therein for investigation and/or treatment separate from the cell culture they were emitted from.

Embodiments are based on the realization that it would be advantageous to provide a fluidic device with the above-mentioned integrated functionalities of cell culturing, cell transportation, and cell filtering for investigation of metastasis of cancerous cell cultures (e.g. a biopsy).

In this context it has been recognized that devices, systems and methods as provided herein provide such functionalities while avoiding the need to pump any media (e.g., fluid carrying emitted cells) off any cell culturing device to a further analysis device using external transport media such as tubes or pipes. Rather, the relevant cells and/or media for assessment can be contained or captured within the same device. This may have one or more of the following advantages: increase ease of analysis, reduction of media volumes used as well as (often expensive) contents thereof, reduction of risk of contamination, and increase analysis accuracy and/or reproducibility due to reduced dead space where emitted cells may be retained. Furthermore, a manufacturing method may be simplified. The integrated substrate setup significantly increases ease and repeatability of manufacturing, thereby increasing consistency across a plurality of different fluidic devices produced in a same way. This also makes test results more comparable and or reliable. Furthermore, integration of multiple pairs of the herein described culturing and filtering chambers in one substrate is made easier and results in a device that can be used in standard, known in the art, analysis equipment for medium to high throughput investigations. The devices may be used for example to speed up search of effective drugs during e.g. personalized treatment of cancers.

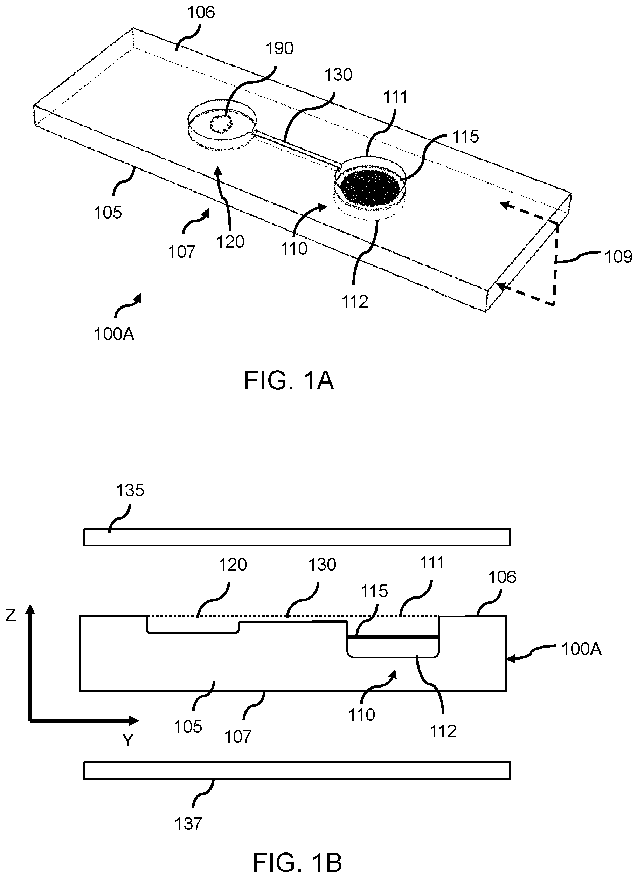

Figs. 1A to 1C illustrate fluidic devices 100A to 100C according to currently disclosed principles. The fluidic devices 100 include: a substrate 105 comprising a first chamber 110 divided by a perforated membrane 115 into a first sub-chamber 111 and a second sub-chamber 112, a second chamber 120, and a first fluid channel 130 connecting the first sub-chamber 111 to the second chamber 120. Any one of these chambers and channel, as well as those of other fluidic devices disclosed herein, can take the form of recesses, holes, cavities, trenches, or the like in the substrate 105.

The substrate 105 is an integrally formed substrate 105 or "monolithic substrate" formed of a single piece of material having the chambers and first fluid channel formed as part thereof the substrate 105 has a first surface 106 and a second surface 107 opposite the first surface 106.

The first and second surfaces of each example substrate are mutually parallel and planar or flat, but this is not needed per se.

The first sub-chamber 110 and the first fluid channel 130 are partly enclosed (i.e partly bound) by the substrate material in that they are open on the side of the first surface 106. The first chambers 110 and the second chambers 120 and first fluid channels 130 are recesses extending from first surfaces 106 into the respective substrates.

In use the fluidic devices 100 may be oriented as desired but preferably are oriented horizontally such that the first surface 106 faces upwards along the indicated Z-direction of a Cartesian coordinate system as shown in Fig. 1B. The first surface 106 may then also be referred to as: "upper surface". The second surface 107 may then face downwards and be referred to as a: "lower surface".

The chambers 110 and 120 each have cylindrical shape with a cylinder axis perpendicular to the first and/or second surfaces 106 and 107. The chambers have circular cross sections (measured perpendicular to the cylinder axis). Chamber depth as measured along the cylinder axis is less than the height of the substrate, but this is not essential as will be described further below. The depth of the second chamber 120 as measured along the cylinder axis between the bottom of the channel 130 and the bottom of the second chamber 120 is chosen such that the second chamber can contain the entire cell culture specimen submerged in culturing fluid while such fluid does not spontaneously drain off through the first fluid channel. For example, the depth may be in the range of 0.5 mm to 2 cm, or 1 mm to 5 mm.

The substrate 105 comprise a perforated membrane 115 parallel to the second surface 107. In this example, the perforated membrane 115 is separately made from the substrate and added to, attached to or otherwise included in the substrate afterwards. In other examples, however the perforated membrane is an integral part of the substrate.

The perforated membrane 115 divides the first chamber 110 into a first sub-chamber 111 and a second sub-chamber 112 such that only the first sub-chamber 111 is connected to the first fluid channel 130. Thus, the first chamber 110 is formed of two sub-chambers 111, 112 separated by a perforated membrane 115. The first sub-chamber 111 may act as an upper sub-chamber 111 and the second sub-chamber 112 may act as a lower sub-chamber 112 in agreement with definition of "upper" for the upper surface and lower surface.

The perforated membrane 115 has a thickness measured perpendicular to the plane it extends in, *i.e.* for example along the Z-direction in Fig. 1B. Preferred thickness is in the range of 5 to 100 µm, but other thickness can be used.

The perforated membrane 115 comprises a plurality of pores. Each pore is wide enough to allow passage of fluid possibly containing nutrients, drugs or other (bio)molecules such as proteins etc. while the pore is narrow enough to prevent passage of target cells. For example, the pore diameter may be 5 µm, but other values as described hereinafter may be used.

Therewith the perforated membrane 115 can filer the target cells larger than the pores from a fluid carrying such target cells, while allowing passage of the fluid and some of the smaller particle contents such as (bio)molecules or other smaller particles.

The second chamber 120 is for culturing a cell culture 190. Thus, the second chamber may be appropriately sized and/or shaped for facilitating the cultivation of a cell culture, a plurality of cell cultures, or tissue by providing appropriate nourishment in the form of a fluid containing nutrients as well as other necessary ingredients.

Examples of cell cultures include mammalian material, biopsy specimens or samples, cultivated growths such as spheroids or organoids and so on. All of these may be cancerous types and/or other types. The diameter of the second chamber 120 may be in the range of 2 cm or less or 1 cm or less or even 0.5 cm or less depending on the cell culture used.

The first fluid channel 130 extends between the first chamber 110 and the second chamber 120 in the form of a recess in first surface 106. In this example the channel is straight with a length of 500 µm, but this is not necessary and other shapes and lengths as described herein after may be chosen. The location of the first fluid channel 130 near surface 106 ensures a suitable depth in the second chamber. The chamber channel connectivity makes it possible to cause a fluid flow in the device from the second chamber via the first flow channel to the first sub-chamber 111 through the permeable membrane 115 to the second sub-chamber 112 by for example adding fluid to the second chamber 120. Therewith cells emitted by cell culture 190 may be transported with such flow to the first sub-chamber 111 and retained there by the perforated membrane 115. Hence their investigation separate from the cell culture may be facilitated.

Many design variations and additional features to the above fluidic devices 100A to 100C and their substrates to assist in one or more functionalities are contemplated some of which will be described below.

The fluidic devices 100A to 100C may have one or more cover plates 135 and 137. The substrates may be permanently, or releasably clamped or adhered to the cover plates. One or more of the cover plates 135 and 137 may comprise or consist of plastic such as polystyrene or polycarbonate or of glass or quartz or other inorganic substrate. They may be glass sheets or slides. Such as microscope slides.

A manner of clamping or adhering is exemplified in Fig. 1B where substrate 105 is stacked with its surfaces 106 and 107 between the first cover plate 135 and second cover plate 137. One or more of these cover plates may provide structural or mechanical integrity (which is advantageous if the substrate is made of elastomeric materials) and/or serve to further enclose (preferably fluid leak free) parts of the first and second chambers and any channels exposed at the surfaces 106. Note that to further enclose the chambers 110 and 120 and first fluid channel 130 of fluidic device 100A and 100B of Fig. 1A only the first cover plate 135 is needed. This may provide also enough structural integrity so that plate 137 is not needed.

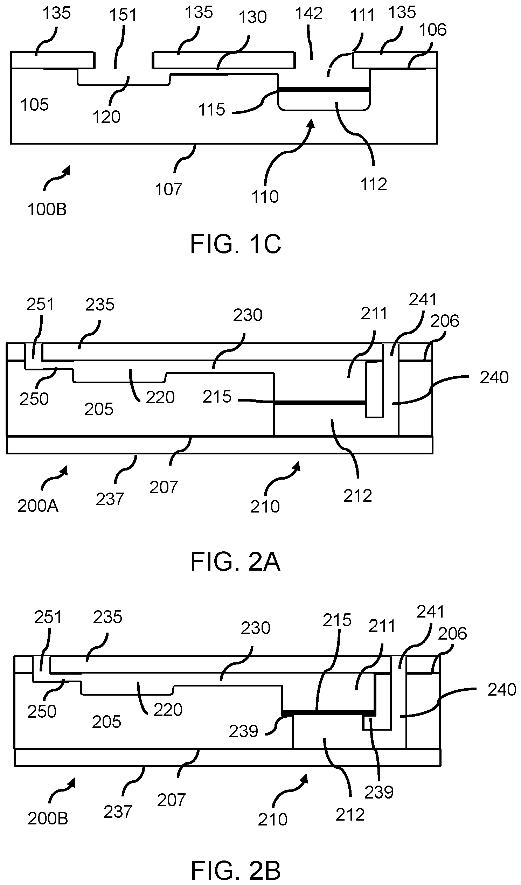

In so called "open fluidic devices", one or more of the first and second chambers and the first fluid channel may be exposed to an outside environment from the surface 106 side allowing access by a user to one or more chambers comprising the cell culture or any emitted and filtered cells. Such access may serve nourishment, treatment, analysis or harvesting of cells, cell cultures etc. The device of Fig. 1A without plate 135 is an example. Another example is device 100C shown in Fig. 1C where cover plate 135 has openings 142 and 151 at locations of the chambers therein. Note that I device 100C the cover plate does still further enclose the first fluid channel 130.

In a so called "closed fluidic device" the chambers and preferably the first fluid channel 130 are enclosed by a first cover 135.

In fluidic devices to be used in both closed and open mode one or more covers and preferably the first cover 135 is temporarily releasable from the substrate if so desired.

Figs. 2A and 2B illustrate fluidic devices 200A and 200B each with a substrate 205. They have parts and features corresponding to some of those described for Figs. 1A to 1C. Corresponding parts have similar reference numbers such that the first digits indicate the figure number and second and third digits indicate corresponding part numbers. For example, parts 105 and 205 refer to corresponding parts. Different parts and features will be described below.

The first chamber 210 extends through the respective substrate 205, *i.e.* its depth is equal to the substrate height. This may be convenient for manufacturing since the perforated membrane 215 can be made as an integral part of the substrate 105 using for example casting, injection molding or 3D printing as described herein after. This is indicated for the device 200A. The fluidic devices 200A and 200B have a second cover plate 237 for structural integrity and to further enclose the second sub-chamber 212 and parts of some channels such as channel 240. The first cover plate 235 may then be present or not and designed according to need to serve the desired enclosure purposes and/or define open or closed fluidic devices as described before.

The substrate 205 comprises a first output fluid channel 240 connecting the second sub-chamber 212 to a fluid outlet 241. The fluid outlet 241 is located at the top surface 206 but may be located elsewhere. The channel 240 allows continued fluid flow for cell filtration or cell capturing. This design allows continued flow in substrate 105 for such purpose may stop due to chamber 212 become filled. The substrate 205 also comprise a first input fluid channel 250 between a fluid inlet 251 and the second chamber 220. The fluid inlet 251 is located at the top of the substrate 205 but may be located elsewhere. The cover plate 235 have openings 251' and 241' (not indicated for clarity) that align with the respective fluid inlet 251 and fluid outlet 241. The devices allow a continued flow of fluid from chamber 220 to second-sub-chamber 212 to filter cells which can be controlled from one side of the fluidic device.

Fig. 2B shows a substrate largely resembling that of Fig. 2A but wherein the perforated membrane 215 has been inserted as a separately made part into the substrate 205. To support the membrane 215, the first chamber 210 of device 200B has a membrane support feature 239. In this case such feature is provided as a rim 266 in the first sub-chamber 211 by designing the diameter of the first sub-chamber to be slightly larger than that of the second sub-chamber 212 so that a support ridge is created for carrying the membrane. Other support features may be used. In general, and applicable to all fluidic devices herein, a separately inserted membrane allows customization of a membrane for purposes independently from manufacture of the rest of the substrate. For example, the membrane material may be differentiated from the substrate material to be better suited for cell culturing.

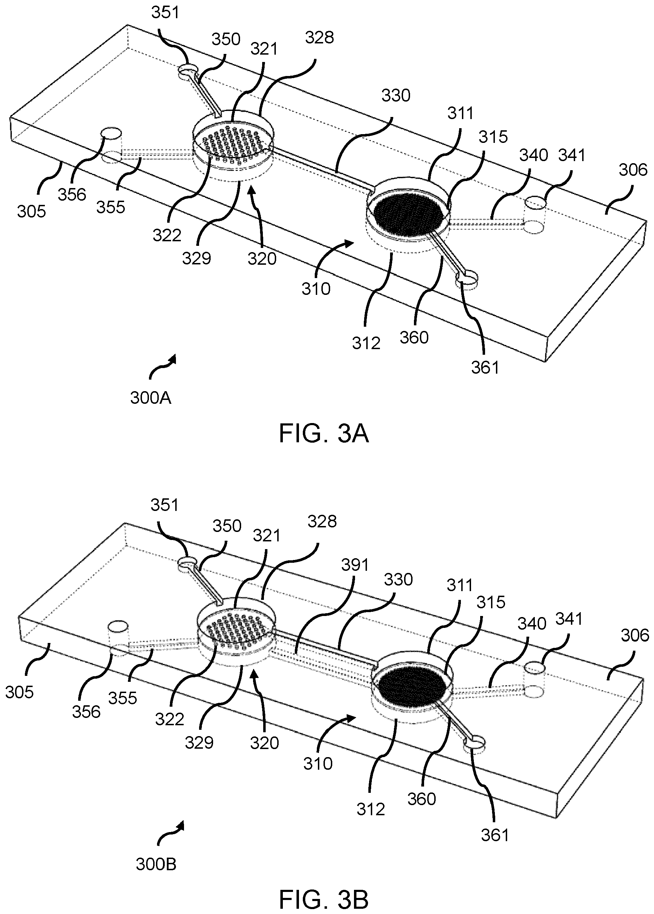

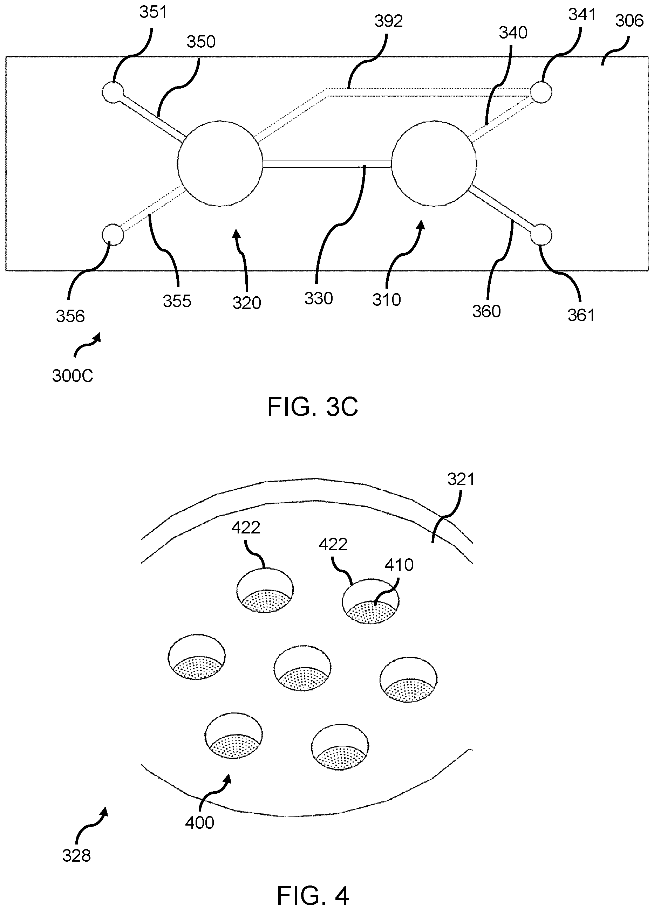

Figs. 3A to 3C illustrate fluidic devices 300A, 300B and 300C according to disclosed principles. Again, these devices have some parts and features corresponding to those of devices 100A, 100B, 200A and 200B. Corresponding parts have similar reference numbers such that the first digits indicate the figure number and second and third digits indicate corresponding part numbers. For example, parts 105 and 305 of respective Figs 1 and 3 refer to corresponding parts.

Thus, the fluidic devices 300A, 300B and 300C again each comprises an integrally formed substrate 305 and, as before, the integrally formed substrate 305 comprises a first chamber 310, a second chamber 320 each extending entirely through the substrate, and a fluid channel 330. The fluidic devices comprise a perforated membrane 315 which separates or divides the first chamber 310 into a first sub-chamber 311 and a second sub-chamber 312. The fluidic device may include one or more cover plates (not shown for clarity) with appropriate openings to match fluid inlets and outlets as described herein before in relation to fluidic devices of Figs. 1 and 2.

Each second chamber 320 is now also divided in two sub-chambers. Thus, there is a third sub-chamber 328 and a fourth sub-chamber 329 with a supporting member 321 in between. The supporting member may be an integral part of the substrate 305 or may be added as a separately manufactured piece, sometimes referred to as "a supporting member insert", just as described before for the perforated membrane herein. The supporting member may be implemented in several ways.

In a first implementation the supporting member 321 comprises or consists of a second perforated membrane 321 which may be embodied as described herein for any other perforated membrane such as the first perforated membrane 315. The two perforated membranes 315 and 321 may then be the same or identical, but this is not essential. Just like the first perforated membrane 315, the second perforated membrane 321 allows fluid flow between the two sub-chambers it separates.

In a second implementation the supporting member 321 comprises one or more (or a plurality of) separate wells 322 which have a size and shape as for example described herein. The wells have their open side towards the third sub-chamber. The supporting member is chosen thick enough to accommodate the wells with a depth of *e.g.* 3000 µm or less, although this is not an essential value.

The supporting member 321 with the wells preferably further includes one or more perforated membranes. Preferably these are present the bottom of one or more of the wells, but this need not be the case. Fig. 4 illustrates a portion of a third sub-chamber 328 having a supporting member 321 that includes 7 wells 422 each of which has a second perforated membrane 410 at its bottom. The membrane thickness and pores may be sized and shaped as defined herein for other perforated membranes. The precise size may be tuned to the cell culture to be cultivated in the well. In this way, the supporting member may provide another example of a second perforated membrane. The thickness and pore dimensions of any membrane in the support member 321 may be, but need not be, identical to those of the first perforated membrane 315.

Having a perforated membrane in the supporting substrate 321 may be desired to provide nourishment and or treatment therethrough to the cell culture in the third sub-chamber while cells emitted by the cell culture cannot enter the fourth sub-chamber.

In yet another implementation of the support member an incision or slit is provided in any supporting member 321 described herein. Preferably such incision or slit is provided as a cut through the whole layer thickness (full cut-through) of the supporting member. A biological specimen may be put between or in the incision by applying an external force to the supporting member. In this way, the supporting member may be configured to support a biological specimen in an incision, i.e., comprise an incision for supporting or holding a biological specimen.

An incision as used herein denotes a slit-shaped opening that, when no external force is applied to the supporting member, leaves a gap between opposing cutting ends of less than 1000 µm, preferably less than 500 µm, more preferably less than 250 µm and most preferably less than 100 µm. The incision has preferably the shape of a straight line or a cross.

By applying an external force to the supporting member 321 the gap is widened to be able to receive a biological specimen. The external force may be applied as a bending force, a pulling force or a pushing force. Due, for instance, to elastomeric properties, the incised supporting member is capable of clamping and/or fixing in place a biological specimen, when no external force is applied to the supporting member.

This approach provides a mechanism for securing supporting a relatively large piece of biological material such as for example a biopsy.

In some examples, the supporting member with slits may have the perforated membrane and or any wells as described herein.

In some examples the supporting member 321 takes the form of a supporting member insert. In such case the second chamber 320 preferably comprises features for supporting such insert. For example, a rim like the rim 239 described for supporting perforated membrane 215 in device 200B may be provided to the second chamber 320. The insert may have similar advantages as described for an inserted perforated membrane.

Suitable examples of inserts with slits are disclosed in the International Patent Application having publication number WO 2021/099213 A1. Any such insert disclosed in this patent application can be integrated into a herein proposed fluidic device, e.g., by positioning such an insert in the second chamber.

Turning back to Figs. 3A to 3C, the first fluid channel 330 of devices 300A to 300C connects the first sub-chamber 311 to the third-sub-chamber 328, such that fluid introduced to the third sub-chamber can transport cells from the third sub-chamber 328 to the first sub-chamber 311 as described before for the first fluid channels of devices of Figs. 1 and 2.

The fluidic devices 300A to 300C each further comprise a first input fluid channel 350 connected to the third sub-chamber 328. The first input fluid channel 350 comprises an exposed portion for receiving fluid for the third sub-chamber 328. The exposed portion of the first input fluid channel 350 may be a first inlet 351. The first input fluid channel 350 may be used for providing fluid for transporting any cells to the first sub-chamber. Alternatively, or additionally, nourishment and/or treatment may be provided to the third sub-chamber using this channel.

Each of the fluidic devices 300A to 300C comprises a second input fluid channel 355 connected to the fourth sub-chamber 329. The second input fluid channel 355 comprises an exposed portion in the form of a second inlet 356. In particular, the second input fluid channel 355 may extend from the fourth sub-chamber 329 for receiving fluid for the second chamber 320. The second input fluid channel may carry cell culture nourishment and/or drugs for treating any cells carried or supported in the third sub-chamber 328. Fluid introduced to the fourth sub-chamber 329 can reach any cells supported in the third sub-chamber 328 via the second perforated membrane 321 or via any incision or slit as described hereinbefore.

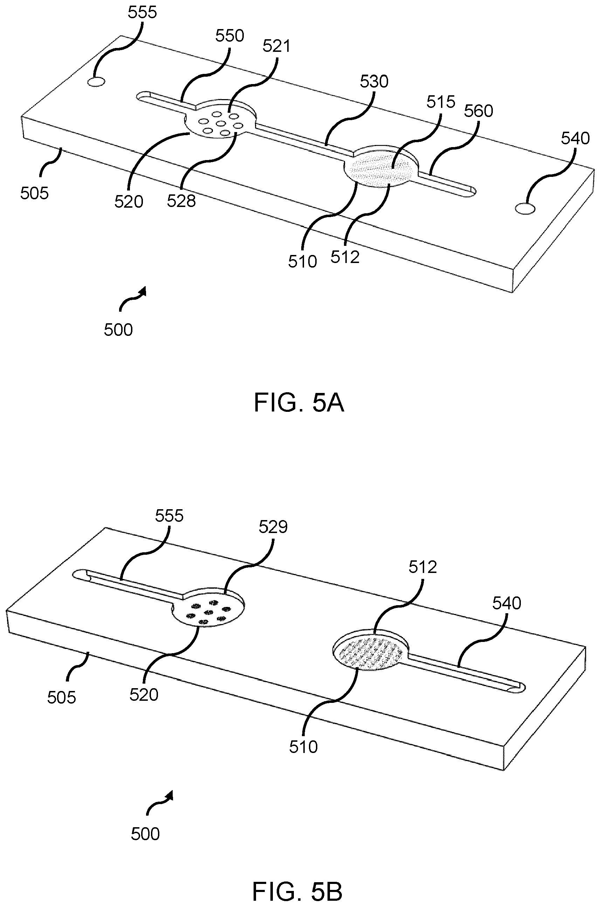

The first and second input fluid channels may be configured such that their respective inlets are at different locations in the substrate. For example, the channels may extend from different angles with respect to the second chamber. This allows both input fluid channels to be exposed to a same side (*e.g.* first surface 306) of the devices for ease of use. Alternatively, to achieve a similar effect, the first and second input fluid channels may extend in a same direction, but be of different lengths (e.g., the second input fluid channel may be longer than the first input fluid channel). The device of Figs 5A to 5D provide an example of such configuration of inlets and input channels.

The fluidic devices 300A to 300C may comprise, a first output fluid channel 340 connected to the second sub-chamber 312 to allow the output or extraction of fluid from the second sub-chamber 312. Therewith the channel 340 allows for a continuous flow of fluid from the second chamber to the first chamber, e.g., for performing longer term nourishment and/or treatment of cell culture and/or metastasized cells in the first sub-chamber.

The first output fluid channel 340 may connect the second sub-chamber to a (external) surface of the substrate 305. For instance, the output fluid channel 340 may, for instance, be partially exposed to the first planar surface 306 of the fluidic device, e.g., to allow the fluid to be extracted. The output fluid channel 340 may be exposed, for instance, at a first outlet 341 for the fluid channel (which connects the surface 306 to the remainder of the output fluid channel 340).

The substrate 305 may further comprise a second output fluid channel 360, sometimes also referred to as "overflow fluid channel". The overflow fluid channel is configured to divert fluid overflow in the first chamber away from the first chamber if needed. On instance of need may be when flow for nourishment and/or treatment is provided to the first and third sub-chambers without such flow having to pass the first perforated membrane. The overflow fluid channel 360 may be exposed, for instance, at a second outlet 361 which connects the surface 306 to the remainder of the overflow fluid channel 360.

If both the fluid channels 340 and 360 are present, they preferably are arranged such that the respective first and second outlets are at different locations in the substrate. For example, the fluid channels 340 and 360 may extend from different angles with respect to the first chamber 310. This allows both fluid channels to be exposed to a same side of the device 300 for ease of use. Alternatively, to achieve a similar effect, the output and overflow fluid channels may extend in a same direction, but be of different lengths (e.g., the overflow fluid channel may be longer than the output fluid channel). Figs. 5A to 5D provide an example of such in line configuration of inlets and outlets.

Fig. 3B illustrates a fluidic device 300B which is a variant of the fluidic device 300A. The fluidic device 300B, in contrast to device 300A, comprises a bridging fluid channel 391 that fluidically connects the second sub-chamber 312 to the fourth sub-chamber 329. This avoids the need for a flow between the first and second chambers to always pass through the perforated membrane(s) to enter or exit the lower sub-chambers (which might cause a blockage or dead end). This can, for instance, permit the extraction or replacement of fluid in the second sub-chamber without said fluid needing to flow through the perforated membrane(s). Also, this extraction feature makes use of an already present first output fluid channel 340 and associated outlet 241.

By providing a bridging fluid channel 391, flow in the lower sub-chambers (second and fourth sub-chambers) can be separately controlled from flow in upper sub-chambers (first and third sub-chambers). Thus there is a so called "upper flow system" including channel 352, sub-chamber 328, channel 330, chamber 311 and channel 360 and a so called "lower flow system" including channel 355, chamber 329, channel 391, chamber 312. The two flow systems are in mutual fluidic connection only via any perforated membranes. This may be advantageous in that the fluid contents of these systems may be separated refreshed, for example for changing treatment while keeping nourishment the same or vice versa. Refreshment fluid can be introduced to the second input fluid channel 355 and extracted via the overflow fluid channel 340 (*e.g.,* via the second outlet 341). The refreshment fluid may flow through the bridging fluid channel 391, through the second sub-chamber or even only the second sub-chamber to the overflow fluid channel 340 without substantially disturbing conditions set in chambers 328 and 311 comprising cell cultures or cells. At the same time the systems may be used to fluidically interact, for example, for transport of cells where fluid from the third sub-chamber may be caused to traverse the first perforated membrane during a cell filtration procedure.

Fig. 3C illustrates fluidic device 300C as another variant of the fluidic device 300A or 300B. The fluidic device 300C comprises a bypass fluid channel 392 that fluidically connects the fourth sub-chamber 329 to the second outlet 341 (or alternatively, but not illustrated, to the overflow fluid channel 360). The bypass fluid channel 392 achieves a similar advantage to that of the bridging fluid channel of device 300B, but is not in direct connection with the second sub-chamber 312. This can reduce a risk of fluid present in the fourth sub-chamber 329 having to flow to the second sub-chamber 312 - e.g., as fluid may be continuously sucked through the second outlet 341. The bypass channel can be implemented to have an outlet that is separate from the second outlet 341 (not shown), but this requires an extra outlet which is sometimes not desired.

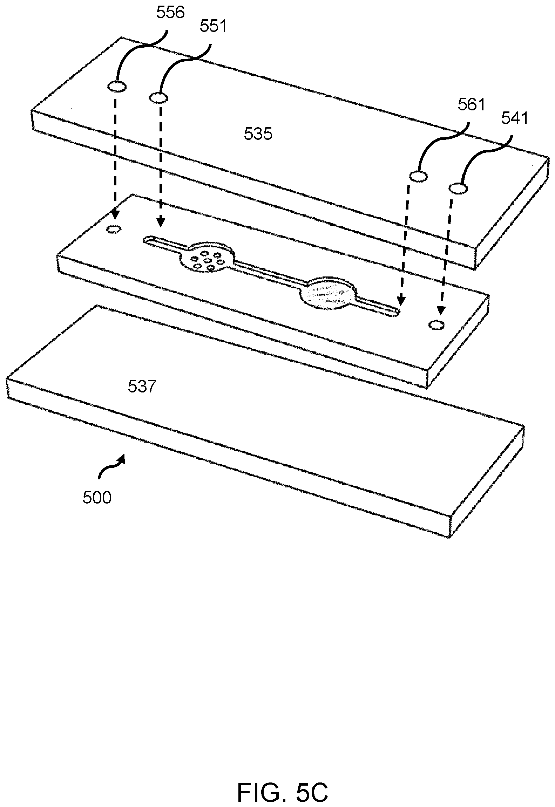

Figs. 5A to 5D illustrate another fluidic device 500 according to principles disclosed herein. Again, these devices have some parts and features corresponding to those of devices 100A, 100B, 200A, 200B, and 300A to 300C. Corresponding parts have similar reference numbers such that the first digits indicate the figure number and second and third digits indicate corresponding part numbers. For example, parts 305 and 505 of respective Figs. 3 and 5 refer to corresponding parts.

The fluidic device 500 comprises an integrally formed substrate 505, i.e., a uniform or monolithic piece of material. As before, the integrally formed substrate 505 comprises a first chamber 510, a second chamber 520 and a fluid channel 530. The device includes within the second chamber 520 a supporting member having the wells with perforated membrane bottoms as described herein before. The supporting member divides the second chamber 520 in a third sub-chamber 528 and a fourth sub-chamber 529. Also, there is a perforated membrane 515 in the first chamber 510 dividing the chamber in a first sub-chamber 511 and second sub-chamber 512. The first perforated membrane may be embodied as described herein before.

The fluidic device 500 differs from the previously described fluidic devices 300A to 300C in that the first 550 and second 555 input fluid channels are not angled with respect to one another and that the first 540 and second output channel 560 (the latter sometimes also referred to as "overflow channel 560") are not angled with respect to one another.

It will be appreciated that the fluidic device 500 may further comprise a bridging fluidic channel and/or a bypass fluidic channel (not illustrated) like those described for devices 300B and 300C.

Thus, in this embodiment, the top and bottom fluid channels overlay one another. This provides a compact configuration. Such configuration is also convenient for implementation in multichamber substrates. Thus, for example, the device 500 may define a dual chamber unit and a multi-dual chamber substrate may be configured such that corresponding input and output channels of the different units are connected to each other. In other words, the units share joint inputs and outputs. This allows for parallel connection of units and parallel operation thereof using a reduced number of inlets and outlets.

Fig. 5C is an exploded view of the fluidic device 500 comprising two cover plates 535 and 537. Any device with one or more cover plates is sometimes also referred to as a "fluidic stack". The fluidic device 500 comprises the substrate 505 of Figs. 5A and 5B, a first cover plate 535 (sometimes also referred to as upper plate) and a second cover plate 537 (sometimes also referred to as a bottom plate). In use, the substrate 505 may be adhered to, pressed against any one of the cover plates, or clamped between the cover plates. This may provide structural integrity or support to the device 500. Clamping may be done using a separate clamping system (not shown). The first cover plate 535 may comprise a plurality of through-holes or apertures 541, 551, 556 and 561 that serve as the first and second inlets and first and second outlets as described before for other example devices. The inlets and outlets line up with the input and output channels along the dashed arrows upon combining the cover plate with the substrate. Thus, each through-hole or aperture may be aligned with a fluid channel to facilitate the provision or extraction of fluid to the chambers via the fluid channel.

In some preferred embodiments, combinations of selections or of all of the optional features set out above are included within a fluidic device 500. For example, a bridging and/or bypass channel may be added. The bridging channel 591 may run between fourth sub-chamber 529 and second sub-chamber 512 in the substrate 505 shown in Fig. 5B.

One embodiment that facilitates ease of use of the fluidic device omits one of the first and second input fluid channels and omits the overflow channel. These fluid channels can be seen as superfluous in some examples, such that their omission can provide a useful fluid device that is more structurally robust and/or compact.

Any one of the fluidic devices disclosed hereinbefore includes a combination of two (a pair of) chambers including the first perforated membrane and a support member if any and any channels extending between the two chambers to provide the desired functions to the device. Such combination may be referred to as a dual chamber unit. In some examples of fluidic devices according to currently disclosed principles there is a plurality of such dual chamber units within a substrate. Thus, any one of the configurations of chambers and channels of a dual chamber unit described herein before may be provided multiple times within a substrate without further modifications. Other multi dual-chamber unit fluidic devices examples have further modifications to provide additional integration between the multiple dual chamber units. For example, and as already described here above, fluid input and fluid output channels of different units may be share. E6xamples will be described below.

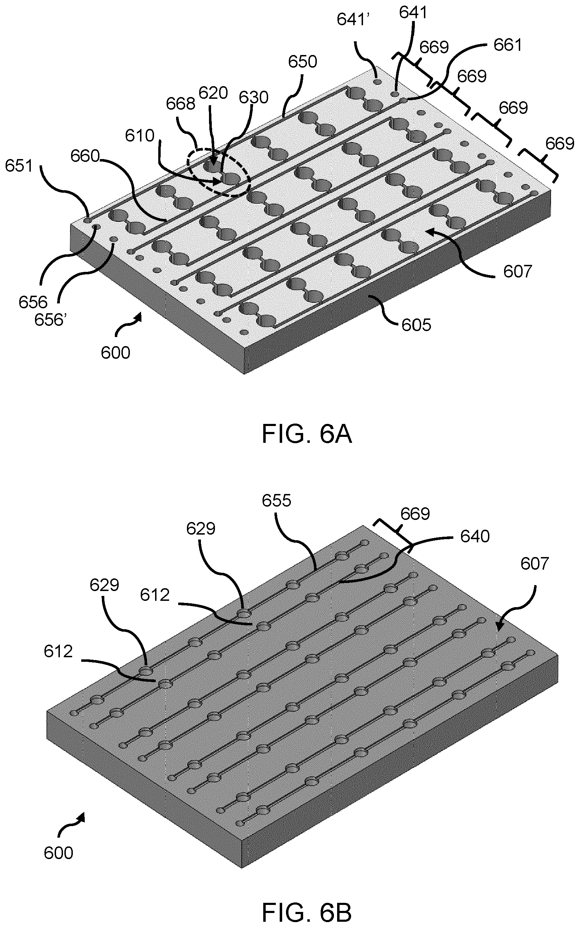

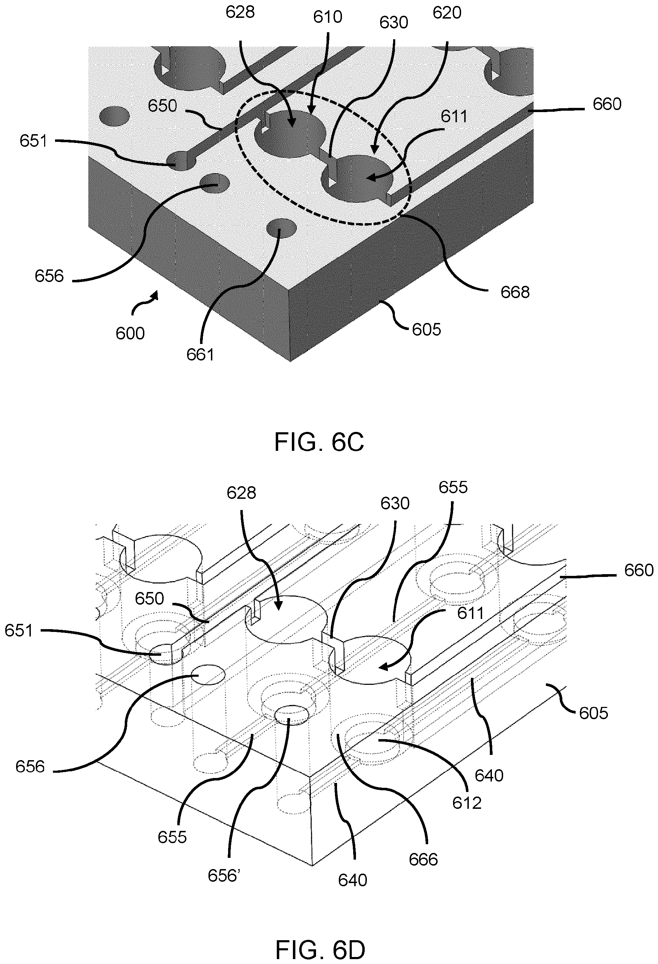

Figs. 6A and 6B show a top and bottom view of a fluidic device 600 with a substrate 605 wherein 24 dual chamber units 668 are provided. Figs. 6C and 6D provide a magnified and transparent view of the same portion of the substrate 605. One of the dual chamber units 668 is indicated within a dashed oval line in Figs. 6A and 6C. Any one of these dual chamber units may be embodied as described hereinbefore according to need and they may be mutually identical or different. In the example shown each of the dual chamber units 668 includes a first chamber 610 and a second chamber 620 connected by a first fluidic channel 630. Not shown for clarity are a perforated membrane 615 within each chamber 610 and a supporting member 621 within each chamber 620. Consequently, each of the chambers 610 is divided in a first sub-chambers 611 and third sub-chamber 628 which can be seen in the view of Figs. 6A and 6C. The second sub-chambers 612 and fourth sub-chambers 629 can be seen in the views of Figs. 6B and 6D. Note that in Fig. 6D the perforated membrane 615 and the supporting member 621 have been omitted for clarity but would be located at and supported by the circular rims 666 within the first and second chambers.

In this case, and this may be used for other fluidic devices too, the rims 666 are positioned in the chambers such that the perforated membrane 615 and supporting member 621 are located such that the second and fourth sub-chambers 629 have a relatively small volume, and in this example a volume smaller than that of the first and third sub-chambers. This may be advantages as the smaller volume sub-chamber flow system can be used to administer expensive drugs for therapy. After all a minimum of such drugs is preferably administered for cost reduction but in fluids with still adequately high concentrations. Cell nourishment is generally less critical in this sense and may then be administered using larger volume flow systems. The smaller volume flow system may be the lower flow system within the fluidic device.

There are 4 mutually independent groups 669 each comprising 6 dual chamber units configured in parallel. Thus, each group has its 6 dual chamber units connected to a joint first input fluid channel 650 connected to a first inlet 651 and a joint second outlet fluid channel 660 connected to a second outlet 661. Furthermore, within each group 669, the 6 second sub-channels 612 are connected in series by a joint first output fluid channel 640 connected between a further second inlet 656' and first outlet 641. The 6 fourth sub-channels 629 within each group 669 are connected in series by a joint second input fluid channel 655 connected between a second inlet 656 and a further first outlet 641'.

This chamber connectivity is advantageous for several reasons. Firstly, it reduces inlets and outlets and does so while between the 4 groups 669 and/or between the second and fourth sub-chambers of a group, the fluid flow and composition can still be independently controlled. Thus, the fluidic device 600 has an upper flow system comprising channels 650, 630, 660 first 611 and third sub-chambers 628 and accessible through inlet 651 and outlet 661 wherein 6 dual chamber units are in parallel flow connectivity. The fluidic device has a first lower flow system comprising 6 fourth sub-chambers 629 fluidically connected in series by the first input fluid flow channel 655 between inlet 656 and further outlet 641'. The fluidic device has a second lower flow system comprising 6 second sub-chambers 612 fluidically connected in series by a second input fluid flow channel 640 between further inlet 656' and outlet 641'. The upper flow system and first lower flow system are fluidically connected to each other via first perforated membranes 615 and the upper flow system and second lower flow system are fluidically connected by the supporting members 621 if these contain anu perforated membranes or other fluid passage.

The upper and first and second lower flow systems may be mutually independently controlled with flow and composition of fluid to create various (same or different) conditions in the cell containing sub-chambers. Thus, for example, the upper flow system may be used to provide the same or similar nutrition for cell cultivation to the cell containing sub-chambers. Also if connected to the second lower flow system it may be used for fluid flow for transportation of cells (e.g. mimicking blood flow in tissue of a subject). The lower flow systems may then be used to provide drug or other treatments via the respective perforated membranes to the sub-chambers housing the cell culture and metastasized cells. Refreshing for all chambers and sub-chambers is possible without dead ends.

As a variation to the connectivity configuration and to further reduce inlets and outlets, inlets 656 and 656' may be connected to each other or integrated as one and alternatively, or additionally the same may be done with outlets 641 and 641'. Alternatively, or additionally two or more of the dual chamber units within a group may have independent first inlet 651 and first input fluid channel 650. The fluidic device has been described with a 4 groups times 6 dual chamber units configuration. Other configurations may be used. Thus, there may be 1 or more groups 669 each having two or more dual chamber units 668. Inlets and/or outlets may be located elsewhere in the substrate or fluidic device.

Preferably any multi dual-chamber unit fluidic device disclosed herein includes a bottom plate (cover plate) 637 (not shown) to enclose the lower flow systems. The bottom plate can be (releasably or permanently) adhered or clamped to the surface 607 shown in Fig. 6B. There may be a cover plate 635 to permanently or releasably cover surface 607 of the substrate 605. No cover plate, a releasable cover plate or a partially open cover plate is beneficial for user access from the top as described herein before for other fluidic devices.

The fluidic device can thus be used as both an open and closed fluidic device by simply removing or adding the cover plate 635.

The fluidic device and its substrate may have a shape and size to correspond to well cell plates well-known and generally used in the field. This allows use of the fluidic devices in existing investigation and analysis equipment. For example, the fluidic device 600 includes a 48 well configuration, each chamber providing a well. The wells may thus have shapes sizes and locations in the substrate to accommodate the above. Another often used configuration is a 96 chamber (96 well) configuration thus having 48 dual chamber units independently usable or for example organized in groups as defined herein. Together with the open configuration the device thus allows operation using pipetting from the top as is often used in the art of cell culturing and/or investigations.

The dimensions of chambers and channels in the multi dual chamber unit fluidic devices may be chosen as described herein before for other fluidic devices. As a maximum chamber diameter 10 or 20 mm may be chosen so that the substrate dimensions may be kept relatively small or multiple units/pairs of first and second chambers may be defined in one substrate. At the same time such channel dimensions provide sufficient space for the capture and subsequent analysis of any cells transported to the first chamber by a fluid flow along the fluid channel.

The perforated membranes and supporting members may be chosen as described herein for other fluidic devices.

The first and second chambers of fluidic devices described herein may be generally cylindrical in shape as described above. This is particularly advantageous from a fluid dynamics perspective, e.g., to mitigate the pooling of fluid in a corner of the chamber and/or with regard to usability in existing analysis equipment. However, other shapes such as square or rectangular cross-section could be used in variants of the proposed embodiments. The chambers of one device do not need to have the same dimensions and/or shape, such may however be appropriate for performing experiments with similar conditions on one substrate.

Any of the substrates herein may be sized and shaped according to need with dimensions such as length, width and height. For multi dual chamber unit devices a typical length and width are in the range of 20 cm or less while a typical height is in the range of 3 cm or less. Preferably length and width are 15 cm or less while height is 2 cm or less. Other dimensions may be chosen however. For single dual chamber units typical length and will may be less such as less than 10 or even 5 cm with thicknesses of less than 1 cm or even less than 5 mm.

Any of the perforated membranes may be configured as follows. A preferred thickness is in the range of 5 to 100 µm, but other thickness can be used. More preferably the thickness is in the range of 5 to 40 µm or of 10 to 40 µm. Most preferably the thickness is in the range of 10 to 20 µm. For thicker membranes pores are more difficult to make e.g. using laser perforation and thinner layers are more difficult to make using injection molding, 3D printing or casting.

The pore diameter in perforated membranes disclosed herein preferably is equal to or less than any one of the following maxima: 10 µm, 9 µm, 8 µm, 6 µm, and 5 µm.

The pore diameter may be tuned to types of cells to be captured. By way of example, if the desired cell (or cell of interest) is a leukocyte (white blood cell), then the maximum pore diameter may be 3 µm or less, e.g., 2 µm or less as these cells can pass through extremely small pores. As another example, cancer cells (of potential interest) are larger than this, and the maximum pore diameter may be set accordingly (e.g. as indicated herein before), to facilitate capture of such cells in the perforated membrane. Hao, Si-Jie, et al. "Size-based separation methods of circulating tumor cells." Advanced drug delivery reviews 125 (2018): 3-20 provides examples of cancer cell sizes that can be used to determine or set a maximum pore diameter, as would be readily understood by the skilled person.

Of course, each pore of the perforated membranes has a diameter sufficiently large to allow the flow of fluid containing constituents such as molecules and proteins therethrough. The theoretical minimum diameter of each pore may therefore be dependent upon fluid type and its contents. For example, for water-based fluids, to ensure sufficient/fast flow of fluid through the perforated membrane, each pore may have a minimum diameter of 1 µm or more preferably 2 µm .

As a working example, if the targeted cell/particle is a cancerous cell emitted from a cancerous biopsy, then each pore of the perforated membrane may have a diameter in the range of 5 µm to 10 µm.

The pore diameter need not be identical for all pores.

The total pore area of the plurality of pores of a membrane is chosen to allow a fluid flow (e.g. in volume per time) that is sufficient to cause transportation of any emitted cells. In some examples, this total pore area is equal to or larger than the cross-sectional area of the first fluid channel. Hence a generated pressure to cause a fluid flow may be kept low even when some of the pores of the perforated membrane become obstructed for fluid flow because of the capturing of cells. A reduced pressure may also prevent rupture of the relatively thin perforated membrane 115.

In general, the second chamber or support member therein may comprise one or more (or a plurality of) separate wells extending into its bottom if there is no supporting member. Each well is configured or usable for culturing a separate cell culture. This facilitates the use of multiple different cell cultures in a same fluidic device, to improve efficiency of assessing the cell cultures.

The number of wells may be any suitable number and can vary depending upon the use-case scenario. For instance, the number of wells may be no less than 5, e.g., no less than 50. Of course, the number and size of the wells will affect the minimum size for the second chamber.

Each well may be appropriately sized and shaped to house or support a particular cell culture. For example, wells may have a cylindrical shape with a circular or other shape cross-section. Each well may have a depth and cross-sectional dimensions such as length and width for e.g. rectangular cross-section or diameter for e.g. circular cross-section. Thus, the shape and size of each well may depend upon the intended cell culture to be cultivated in the well. As a working example, each well may have a depth in the range of 50 µm to 1000 µm, preferably in the range of 50 µm to 500 µm and cross sectional dimensions (e.g. length and width or diameter) in the range of 0.5 µm to 1000 µm. Wells having cross-sectional dimensions in the range of 0.5 µm to 200 µm may be desirable for culturing cell cultures in the form of spheroids. Wells having cross-sectional dimensions in the range of 200 µm to 1000 µm may be desirable for cultivating organoids. Matching dimensions of wells to the cell cultures localizes the small cell cultures making their analysis easier as the location of the wells is known. Further, during transportation fluid flow the cell cultures are less likely to be removed from the second chamber.

As a working example, a second chamber can comprise 7 wells (as shown in Fig. 4) of 0.8 mm in diameter would benefit from a second chamber having a diameter of at least 4 mm. As another working example, the chamber comprises 57 wells of 0.2 mm in diameter would benefit from a second chamber having a diameter of at least 4 mm. These are non-exhaustive examples for the number and/or size of the wells.

If present, any supporting member thickness may be in the range of 10 µm to 3000 µm, preferably in the range of 10 µm to 2000 µm and more preferably in the range of 10 µm to 1000 µm. The thickness of the supporting member and depth of any wells therein, if any, are preferably chosen such that the part of the well having a perforated membrane section, such as for example the bottom of the well in Fig. 4, has a thickness as defined herein for the perforated membranes.

Any first fluid channel herein may be straight or otherwise shaped, such as curved or meandering. Preferably a fluid channel is 500 µm or longer. This may help to keep any fluid contained in the first and second chambers separate from one another. The fluid channel may have a cross sectional area and shape, defined in a plane perpendicular to the channel length direction. The cross-sectional area is chosen to be larger than the cross section of a cell or a cluster of cells to be transported to reduce clogging of the channel. The cross section may be uniform or may vary along the fluid channel length, for example, it may increase upon going from the second chamber to the first chamber. The cross-sectional area may have a rectangular shape with an equal width and height. Other shapes and areas may be used.

In the examples shown there is one single first fluid channel between the second chamber and the first sub-chamber. In other examples the first fluid channel may however comprise a plurality of sub-first-fluid channels. For example, each of the plurality of channels may fluidically connect the second chamber with the first chamber. Otherwise, the channel may comprise one or more branches.

Cover plates may be sized according to the substrates they cover. Their thickness may be in the range of 5 mm or less, preferably 2 or even 1 mm or less.

The perforated membrane preferably is placed and/or formed such that real-time or posttreatment imaging or optical inspection through the perforated membrane is enabled. In particular, the perforated membrane may be positioned in an optical window or aperture of the substrate and formed of a transparent or translucent material. This means that any cell cultures or cells on either side of the membrane can be observed. The cover plates may be transparent for this purpose.

In any herein described embodiment, as the fluidic device is for use in culturing, analyzing and/or assessing a cell culture and the substrate needs to be manufacturable as an integrated substrate, it would be desirable if the materials of the fluidic device (such as, where relevant, any perforated membrane and/or any supporting member and/or any insert) has one or more of the following properties; is food contact approved; is transparent and/or translucent (e.g., has a visible transmittance of more than 0.8); facilitates appropriate gas exchange to maintain a dedicated pH value of a cell culture medium and/or is capable of maintaining a temperature of 37.0C (e.g., without deforming or breaking). Example materials that can meet one, more or all of these goals include elastomeric materials are provided herein below.

The proposed fluidic devices design as described herein is relatively easily and/or reliably manufacturable using a method with a limited number of well controllable steps. The method at least comprises a step for forming the substrate as disclosed herein using a casting, or an injection molding, or a 3D-printing method. Preferably the injection molding method is used as that allows relatively increased throughput times. Any of the substrates produced is then optionally combined with a desired number of cover plates and one or more perforated membranes and support members, if any. The cover plates may be made using standard techniques for making glass plates or plastic plates. For example, injection molding may again be used to produce plastic cover plates.

In one example method the substrates are manufactured in a single-step using casting, or injection molding or 3D-printing method. Thus, the complete substrate, including any chambers, channels, perforated membranes, supporting membranes and other recesses if any, are made of one material and in one integral step.

However, in some cases the perforated membranes of a substrate when to be formed as integral part of the substrate may comprise pore openings small enough to be difficult to form reliably using any of the single-step methods. In that case the method can have more steps. In the first step a substrate precursor that includes any chambers, channels, and non-perforated base structures for perforated membranes and supporting membranes, if any, as well as other desired recesses if needed is made using a casting, or an injection molding, or a 3D-printing method. The preferred method is injection molding. In a further step one or more, preferably a plurality of, pores are provided in the base structures at the desired locations to form the perforated membranes and therewith the integrally formed substrate. Preferably such pores are provided using a laser ablation or laser drilling process.

In a variation to the two-step procedure, the first step includes the preparation of the precursor substrate by removing material (e.g., by etching such as laser etching) from a slab of material of desired shape to create the required chambers, channels, perforated membranes, supporting members or other recesses where present.

Another approach is to form any perforated membrane and/or supporting member separately from an integrated substrate. Thus, in a first step of a manufacturing method an integrated substrate is formed having the required chambers and channels as well as other features (e.g. rims or other features) for supporting any to be added perforated membranes and/or supporting members, using a casting, injection molding or 3D printing method. The perforated membrane is separately made before being added, coupled or secured to the integrated substrate to result in the fluidic device. This may provide somewhat more flexibility in materials choice of fluidic devices described herein. Thus the perforated membranes and supporting members may be made of a materials (e.g. elastomers) different from the substrate material allowing to tune properties to improve cell culturing properties, transparencies necessary for analysis etc. It enables fluidic devices that provide a more flexible investigation platform. Furthermore, since perforated membranes are relatively thin these are preferably made from elastomeric materials so that they may be made using the preferred injection molding methods.

Various approaches for manufacturing a perforated (e.g., porous) membrane and, in particular, elastomeric perforated membranes are disclosed in the US Patent Application having publication number US2022/228108A1 or WO2023/046744 which are incorporated by reference in their entirety. The latter document also describes methods for forming pores using laser drilling in elastomeric perforated membranes. Preferably the methods are used to prepare pores in elastomeric perforated membranes improved cell culturing such as modified polysiloxane elastomers.

Any described methods for manufacturing the perforated membrane can be incorporated into a proposed method for manufacturing the fluidic device.

Yet, in another example the substrate is manufactured using a 3D printing technique. This will produce a substrate formed of a single uniform piece of material, but is less scalable than an injection molding technique.

The substrate material may be made of any thermoplastic or elastomeric material. When injection molding techniques are used for substrates that include a perforated membrane and optionally also a support member, as an integral part having a membrane, such substrate, or precursor substrate therefore is preferably made of an elastomeric material. The flow properties of such materials allow thin membranes to be made in this way for example using the one or two step method described hereinbefore. Alternatively, when the perforated membrane and optionally also a support member are manufactured as separate pieces, the substrate may be made of other materials such as thermoplastics etc. which may be formed and shaped using known casting, injection molding or 3D printing methods. The perforated membranes and, optionally the supporting members may then still be made of the elastomeric materials.

Suitable elastomeric materials for perforated membranes, supporting members and substrates are based on polysiloxane (such as for example polyalkylsiloxane, polybutadiene rubber (PBR), styrene-ethylene-butadiene-styrene elastomer (SEBS), thermoplastic polyurethane (TPU), and thermoplastic silicone vulcanizate (TPSiV). Others may be used too. Preferred elastomeric materials for are based on polysiloxane which is sometimes referred to as "silicone" based elastomer. Such elastomers include polyalkylsiloxane and polydimethylsiloxane (PDMS) based polymers with or without modifications to improve cell culturing properties.

Particularly desired silicone based elastomers are described in for example WO2019015988A1 and WO2021/058657. The modified silicone elastomers, such as for example the carboxylic acid modified elastomers, disclosed therein may be conveniently used in an injection molding process as described therein. They may be perforated using laser drilling using methods described in WO2023/046744.

Another reason for the elastomeric materials to be used for the substrate is that glass or plastic cover plates can be adhered to the substrates almost leak free and without complicated adhesion materials such as glue. This is also useful to implement the releasable top cover plates of fluidic devices.

The proposed fluidic devices may be designed for use with optical inspection equipment, such as visible light microscopes with or without fluorescence staining. To assess the outcomes of any staining experiments, polysiloxane based elastomers are again particularly advantageous as they show little autofluorescence. Thus, in some preferred examples, the substrate and/or other features of the fluidic device may be formed from a siloxane based elastomer such as those described herein before.

The following exemplary commercially available elastomeric/soft materials (which are transparent or translucent and are food contact approved) can be used, in certain embodiments, as the material for the integrally formed substrate or other features of the fluidic device: Medalist MD-53253 (TPE Teknor Apex); Medalist MD-53273 (TPE Teknor Apex); Mediprene 500602 M-03 (TPE Hexpol); Texin Rx T85A (TPU Covestro); BioSpan^{®} (F SPU | PUR); BioSpan^{®} (SPU | PUR); BJB Polyurethane F-116 A/B | TSU; BJB Polyurethane F-126 A/B | TSU; BJB Polyurethane F-131 A/B | TSU; BJB Polyurethane M-3115 REV 1 A/B | TSU; BJB Polyurethane M-3125 A/B | TSU; CELLENE MC2248 | TPE; CELLENE MC2265 | TPE; CELLENE MC3038 | TPE; CELLENE MC3050 | TPE; CELLENE MC3061 | TPE; CELLENE MC3226 | TPE; CELLENE MC3239 | TPE; CELLENE MC3261 | TPE; Dynaflex^{™} G2706-1000-00 | TPE; Dynaflex^{™} G2711-1000-00 | TPE; Elastocon^{®} 2860L | TPE; Filter-bond^{™} E-3264 | TS; FLEXCHEM^{™} 3551-02 | PVC, Flexible; FLEXCHEM^{™} 4051-02 | PVC, Flexible; FLEXCHEM^{™} 4551-02 | PVC, Flexible; FLEXCHEM^{™} 5051-02 | PVC, Flexible; FLEXCHEM^{™} 5551-02 | PVC, Flexible; FLEXCHEM^{™} 6051-02 | PVC, Flexible; FLEXCHEM^{™} 6551-02 | PVC, Flexible; Medalist^{®} MD-12130 | TPE; Medalist^{®} MD-12130H | TPE; Medalist^{®} MD-12140 | TPE; Medalist^{®} MD-12140H | TPE; Medalist^{®} MD-12150 | TPE; Medalist^{®} MD-12150H | TPE; Medalist^{®} MD-12f150S | TPE; Medalist^{®} MD-12160 | TPE; Medalist^{®} MD-12160H | TPE; Medalist^{®} MD-12170 | TPE; Medalist^{®} MD-12170H | TPE; Medalist^{®} MD-12243 | TPE; Medalist^{®} MD-12337 | TPE; Medalist^{®} MD-12340 NAT | TPE; Medalist^{®} MD-12342 | TPE; Medalist^{®} MD-12344 | TPE; Medalist^{®} MD-12350 | TPE; Medalist^{®} MD-12352 | TPE; Medalist^{®} MD-12362 | TPE; Medalist^{®} MD-125 | TPE; Medalist^{®} MD-130 | TPE; Medalist^{®} MD-13240 | TPE; Medalist^{®} MD-135 | TPE; Medalist^{®} MD-145 | TPE; Medalist^{®} MD-155 | TPE; Medalist^{®} MD-17365 | TPE; Medalist^{®} MD-225 | TPV; Medalist^{®} MD-32045 | TPE; Medalist^{®} MD-32245 | TPE; Medalist^{®} MD-36048 | TPE; Medalist^{®} MD-37063 NAT | TPE; Medalist^{®} MD-42245 XRD1 | TPE; Medalist^{®} MD-42245 XRD3 | TPE; Medalist^{®} MD-74357 XRDll TPE; Medalist^{®} MD-74357 XRD2 | TPE; Mediprene^{®} 500120M | TPE; Mediprene^{®} 500200M | TPE; Mediprene^{®} 500250M | TPE; Mediprene^{®} 500300M | TPE; Mediprene^{®} 500350M | TPE; Mediprene^{®} 500400M | TPE; Mediprene^{®} 500434M | TPE; Mediprene^{®} 500450M | TPE; Mediprene^{®} 500484M | TPE; Mediprene^{®} 500520M | TPE; Mediprene^{®} 500534M | TPE; Mediprene^{®} 500584M | TPE; Mediprene^{®} 500600M | TPE; Mediprene^{®} 500634M | TPE; Mediprene^{®} 500650M | TPE; Mediprene^{®} 500684M | TPE; Mediprene^{®} 500700M | TPE; Monprene^{®} RG-10160H | TPE; ProvaMed^{®} TPE 1120 | TPE; ProvaMed^{®} TPE 1160 | TPE; RABALON^{®} PJ4300C | TPE; RABALON^{®} PJ5300C | TPE; RABALON^{®} PJ6300C | TPE; RABALON^{®} PJ7300C | TPE; SkinFlex 15 F-115 A/B | TSU; SkinFlex BR-60; BRUSHABLE A/B | TSU; T-Blend^{®} TPE-F22 | SEBS; THERMOLAST^{®} M TM3LFT (Series: MC/LF) | TPE; THERMOLAST^{®} M TM3MED (Series: MC/tl) | TPE; THERMOLAST^{®} M TM3RST (Series: MC/RS) | TPE; THERMOLAST^{®} M TM4LFT (Series: MC/LF) | TPE; THERMOLAST^{®} M TM4MED (Series: MC/tl) | TPE; THERMOLAST^{®} M TM4RST (Series: MC/RS) | TPE; THERMOLAST^{®} M TM5LFT (Series: MC/LF) | TPE; THERMOLAST^{®} M TM5MED (Series: MC/tl) | TPE; THERMOLAST^{®} M TM6LFT (Series: MC/LF) | TPE; THERMOLAST^{®} M TM6MED (Series: MC/tl) | TPE; THERMOLAST^{®} M TM7LFT (Series: MC/LF) | TPE THERMOLAST^{®} M TM7MED (Series: MC/tl) | TPE; UNISOFT SPECIAL^{™} DS-35A-CL-M-01 | SEBS; UNISOFT SPECIAL^{™} DS-55A-CL-M-01 | SEBS; Versaflex^{™} G2705 N | TPE; Versaflex^{™} HC 1100-40 Translucent EU | TPE; Versaflex^{™} HC 1348 Natural | TPE; Versaflex^{™} HC MT317 | TPE; Versaflex^{™} HC MT555 | TPE; Versaflex^{™} OM 1040X-1 | TPE; CELLENE MC2248 | TPE; CELLENE MC2265 | TPE; CELLENE MC3038 | TPE; CELLENE MC3050 | TPE; CELLENE MC3061 | TPE; CELLENE MC3226 | TPE; CELLENE MC3239 | TPE; CELLENE MC3261 | TPE; ChronoPrene^{™} 25A | TPE; ChronoPrene^{™} 40A | TPE (CardioTech International, Inc.); Dryflex^{®} 500300S | TPE; Dryflex^{®} 500350S | TPE; Dryflex^{®} 500400S | TPE; Dryflex^{®} 500450S | TPE; Dryflex^{®} 500500S | TPE; Dryflex^{®} 500550S | TPE; Dryflex^{®} 500600S | TPE; Dryflex^{®} 500650S | TPE; Dryflex^{®} 500700S | TPE; Dynaflex^{™} G2701-1000-02 | TPE; Dynaflex^{™} G2706-1000-00 | TPE; Dynaflex^{™} G2709-1000-00 | TPE; Dynaflex^{™} G2711-1000-00 | TPE; Dynaflex^{™} G2712-1000-02 | TPE; Dynaflex^{™} G2730 | TPE; Dynaflex^{™} G2755-1000-00 | TPE; Dynaflex^{™} G2755C | TPE; Dynaflex^{™} G6713-0001 | TPE; Dynaflex^{™} G6713C | TPE; Dynalloy^{™} GP 7810-60T | TPE; Dynalloy^{™} GP 7810-70T | TPE; Dynalloy^{™} OBC8200-BT50 | TPE; Estane^{®} 58123 TPU | TPU-Polyether; Evoprene^{™} 019 | SBS; Evoprene^{™} G 925 | SEBS; Evoprene^{™} G 936 | SEBS; Evoprene^{™} G 942 | SEBS; Evoprene^{™} G 958 | SEBS; Evoprene^{™} G 966 | SEBS; Evoprene^{™} G 967 | SEBS; Evoprene^{™} G 968 | SEBS; Evoprene^{™} G 969 | SEBS; Evoprene^{™} G 970 | SEBS; Evoprene^{™} GC 5685 | SEBS; Evoprene^{™} GC 5686 | SEBS; Evoprene^{™} GC 5687 | SEBS; Evoprene^{™} GC 5688 | SEBS; Evoprene^{™} GC 5689 | SEBS; Evoprene^{™} GC 5690 | SEBS; GLS 422-126 | TPE; GLS 458-140 | TPE; GLS 458-141 | TPE; GLS 458-142 | TPE; K-Prene HYFLEX HF 15 | MPR; K-Prene HYFLEX HF 20 | MPR; K-Prene HYFLEX HF 25 | MPR; K-Prene HYFLEX HF 30 | MPR; Medalist^{®} RG-38052 XRD1 | TPE; megol^{®} PUG 10 | SEBS; megol^{®} PUG 60 | SEBS; megol^{®} TA 60 | SEBS; Monprene^{®} RG-10130 | TPE; Monprene^{®} RG-10140 | TPE; Monprene^{®} RG-10150 | TPE; Monprene^{®} RG-10160 | TPE; Monprene^{®} RG-10170 | TPE; Monprene^{®} RG-15130 | TPE; Monprene^{®} RG-15140 | TPE; Monprene^{®} RG-15150 | TPE; Monprene^{®} RG-15160 | TPE; Monprene^{®} RG-15170 | TPE; Monprene^{®} RG-18240 | TPE; Monprene^{®} RG-18250 | TPE; Monprene^{®} RG-18260 | TPE; Monprene^{®} RG-18270 | TPE; Monprene^{®} RG-19221 NAT | TPE; Monprene^{®} RG-19255 | TPE; Monprene^{®} RG-20140 | TPE; Monprene^{®} RG-20160 | TPE; Monprene^{®} RG-20170 | TPE; Monprene^{®} RG-29068 NAT | TPE; Monprene^{®} RG-29240 XRD1 | TPE; RABALON^{®} MJ4300C | TPE; RABALON^{®} MJ5302C | TPE; RABALON^{®} MJ6301C | TPE; RABALON^{®} MJ7301C | TPE; RAYPRENE^{®} NB221-S4050 | TPE; RAYPRENE^{®} NB221-S4051 | TPE; RAYPRENE^{®} NB221-S4052 | TPE; RAYPRENE^{®} NB221-S4053 | TPE; tefabloc^{®} TO 132 | TPE; Telcar^{®} TL-83-F943D22-NT BLU | TPE; THERMOLAST^{®} K TF2CGT (Series: FC) | TPE; THERMOLAST^{®} K TF3BTL (Series: FC/AP) | TPE; THERMOLAST^{®} K TF3CGT (Series: FC) | TPE; THERMOLAST^{®} K TF3STE (Series: FC/CS) | TPE; THERMOLAST^{®} K TF4AAB (Series: FC/HE/tl) | TPE; THERMOLAST^{®} K TF4BTL (Series: FC/AP) | TPE; THERMOLAST^{®} K TF4CGT (Series: FC) | TPE; THERMOLAST^{®} K TF4STE (Series: FC/CS) | TPE; THERMOLAST^{®} K TF5AAC (Series: FC/HE/tl) | TPE; THERMOLAST^{®} K TF5BTL (Series: FC/AP) | TPE; THERMOLAST^{®} K TF5CGT (Series: FC) | TPE; THERMOLAST^{®} K TF5STE (Series: FC/CS) | TPE; THERMOLAST^{®} K TF5WHA (Series: DW/H) | TPE; THERMOLAST^{®} K TF6AAF (Series: FC/HE/tl) | TPE; THERMOLAST^{®} K TF6BTL (Series: FC/AP) | TPE; THERMOLAST^{®} K TF6CGT (Series: FC) | TPE; THERMOLAST^{®} K TF6STE (Series: FC/CS) | TPE; THERMOLAST^{®} K TF6WCS (Series: DW/CS) | TPE; THERMOLAST^{®} K TF6WHA (Series: DW/H) | TPE; THERMOLAST^{®} K TF6WHB (Series: DW/H) | TPE; THERMOLAST^{®} K TF7AAC (Series: FC/HE/tl) | TPE; THERMOLAST^{®} K TF7BTL (Series: FC/AP) | TPE; THERMOLAST^{®} K TF7CGT (Series: FC) | TPE; THERMOLAST^{®} K TF7WHB (Series: DW/H) | TPE; Topolymer^{®} 8201-B | TPE; Versaflex^{™} FFC 2882-50 EU | TPE; Versaflex^{™} FFC 2882-50 | TPE; Versaflex^{™} G2708 N | TPE; Versaflex^{™} GP 2810-20N | TPE; Versaflex^{™} GP 2810-30N | TPE; Versaflex^{™} GP 2810-40N | TPE; Versaflex^{™} GP 2810-50N | TPE; Versaflex^{™} GP 2810-60N | TPE; Versaflex^{™} GP 2810-70N | TPE; Cawiton^{®} MT920 | SEBS; Cawiton^{®} MT930 | SEBS; Cawiton^{®} MT940 | SEBS; Cawiton^{®} MT950 | SEBS; Cawiton^{®} MT960 | SEBS; Cawiton^{®} MT970 | SEBS.

The perforated membrane and/or supporting membrane may be made of an elastomeric material as described herein above. It may be made of the same elastomeric material as the substrate, for example when both the substrate and membrane and/or supporting member are made of one single piece of material. Again, a preferred material comprises or consists of a polysiloxane based elastomer such as the polyalkylsiloxane (e.g. PDMS) based elastomer.

As the proposed fluidic devices may be designed for use with optical inspection equipment, such as e.g. visible light microscopes, with or without fluorescence staining of cells and cell cultures, (poly)siloxanes are particularly advantageous for forming the material of the perforated membrane and/or supporting membranes as they show little autofluorescence.

The cover plates described herein may be formed of any suitably transparent material, such as glass, PVC or a transparent polymer, such as a cyclic olefin polymer (COP) or a cyclic olefin copolymer (COC), polycarbonate or polystyrene. In case the substrate is made of elastomeric material, one or more of the cover plates is stiffer than the substrate to provide increased structural integrity.

The combination of cover plates as described with elastomeric material substrates is advantageous as it allows good mutual contact (often by sticking together) increasing leak free closure of chambers and channels and therewith fluidic devices. The sticking forces are most often low enough to be able to releasable attach or adhere the cover plates to the substrates involved.

The fluidic devices disclosed herein provide devices for use in several protocols or methods of analyzing cell culture behavior under various circumstances of culturing and therapy to analyze cell culture therapy response including cell emission response and response of emitted cells to therapy. In use, a cell culture will be held in the second chamber and preferably the third sub-chamber of the second chamber if present. After culturing and optional treatment, a transport fluid flow may be used to carry emitted cells to the first sub-chamber to be retained by the perforated membrane. The cell culture and/or retained cells can then be analyzed (e.g. to discover whether dead or alive) or further cultured and/or treated with therapy. Analysis of cell culture and or emitted cells may be done using a microscope or other imaging methodology separate from each other. This facilitates simulation of cell emission without the need to pump any media off-chip. This increases an ease and speed of analysis, as well as reducing any potential impurities or introduction of undesirable foreign media to a simulated treatment outcome.

In some use case scenarios, before the transport fluid is introduced to the second chamber, the cell culture undergoes a therapy or treatment (e.g., exposure to a drug, therapeutic fluid or the like). This allows the effect of therapy or treatment on cell emission quantity and/or quality to be readily assessed. The transport fluid may comprise a therapeutic fluid including a drug. This can allow, for instance, for monitoring or assessment of longer-term exposure to the therapeutic fluid. Also flow characteristics of blood may be mimicked to investigate cell transportation behavior. The devices and methods may thus benefit investigation and treatment identification of e.g., cancerous cell cultures.

It is further noted that any cells could be easily harvested from the perforated membrane or first chamber by disassembling the device. For example, the relevant cover plates for allowing access to the cell culture or cell containing chambers may be absent, locally open, or removable if such plates are designed to close of one or more of the first and second chambers on upper or lower side.

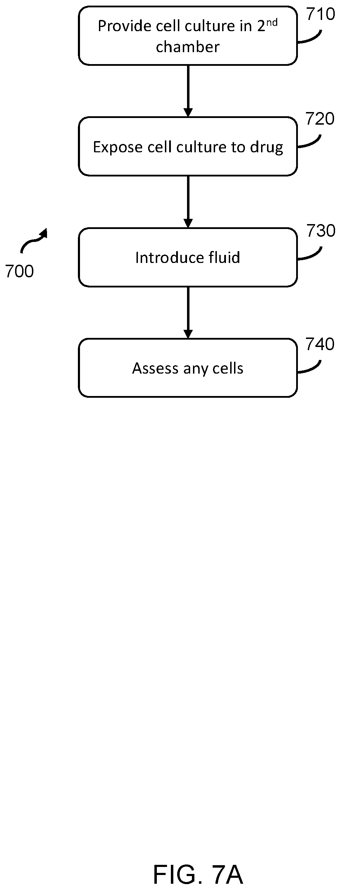

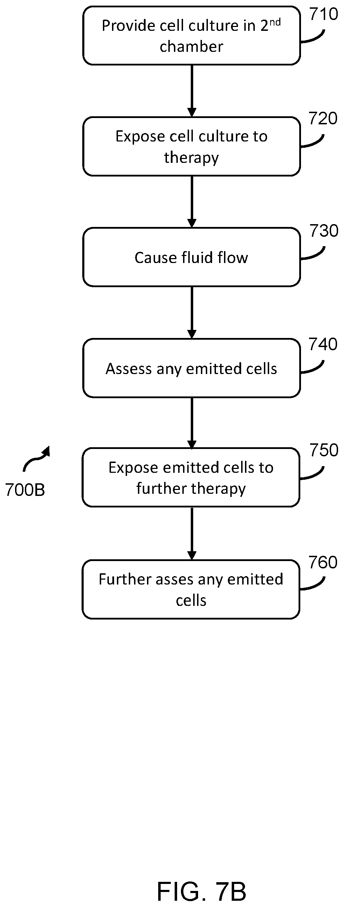

Fig. 7 illustrates a use of fluidic devices described herein in the form of an exemplifying drug testing method 700A. Any fluidic device as described herein may be used in the method. However, the method will be explained with reference to Fig. 1 by way of example.

The method starts with step 710 in which a cell culture is provided to the second chamber of a fluidic device. Thus, for example, the cell culture 190 is provided to the second chamber of device 100 in Fig. 1A. In other devices having a supporting member such as devices 300 and 500 of Figs 3 and 5, respectively, such sample can be provided to the third sub-chamber. The cell culture may, for instance, comprise a previously harvested biopsy sample. Alternatively the cell culture may comprise a spheroid or organoid to be positioned in a well housed in the supporting member of e.g. devices 300 and 500.

The method then comprises a step 720 of exposing the cell culture to a drug. This may be performed, for instance, by providing the drug in the form of a therapeutic fluid such as a chemotherapy fluid that is or carries the drug for exposure to the cell culture. For example, such drug can be provided directly to the second chamber of the device 100. In some examples, the drug is introduced via an inlet and input fluid channel (where present). Thus, for example such drug can be provided via the first and or second inlet of for example fluidic devices 300 and 500.

Step 730 after or during drug exposure step includes introducing a fluid to the second chamber such that the introduced fluid flows from the second chamber, through the first fluid channel and through the perforated membrane in the first chamber. The passage of the fluid through the first perforated membrane may be under the influence of gravity. Alternatively, the force or pressure of fluid flow may encourage or force fluid flow through the perforated membrane. If any were formed, metastasized cells may be carried by the fluid flow and retained within the first sub-chamber of the first chamber by the first perforate membrane. Step 730 may be carried out, for instance, using a fluid pump such as a microfluid pump. Approaches for using a fluid pump to introduce a fluid to a fluidic device are well established in the art and could be repurposed for use with the proposed fluidic device.

Steps 720 and 730 may be combined if, for instance, the drug is carried or formed in/of a fluid.

The method 700A also comprises a step 740 of assessing any cells captured by the perforated membrane. This can be performed using any known cell assessment technique, e.g., using observations through a microscope, using dissection techniques to cut the fluidic device to analyze the captured cells, performing one or more chemical tests on any captured cells, and so on.

Fig. 7B shows a further method 700B. It has steps identical or corresponding to that of Method 700A. However, after step 740, or even after step 730 there is a step 750 in which emitted cells are exposed to therapy such as that described for step 720. The therapy may be the same or different for that applied in step 720 to the cell culture.

After step 750 a step 760 is performed in which the emitted cells are assessed or again assessed, using methods as described for step 740. The assessment may be different or the same as that performed in step 740.

A wide variety of different cell assessment techniques are known in the art, and the selection of any particular cell assessment technique is dependent upon the particular use case scenario or assessment goal. Such techniques may comprise staining techniques for example as known in the art. They may be harvested for further investigation such as DNA sequencing using known in the art techniques etc.

Cells captured in or on the perforated membrane can be subsequently analyzed using external analysis systems. For example, optical investigation using e.g. a microscope is envisaged. If such analysis takes place from the bottom side of the substrate, the perforated membrane is preferably at least to some extent transparent to any radiation with which the cells are analyzed. For example, the at last some extent of transparency may pertain to radiation in the form of light in one or more wavelength ranges of ultraviolet, visible and near-infrared radiation. Add some wavelength ranges for the transparency?? Preferably the membrane is transparent for visible light allowing staining experiments as known in the art to be performed on the captured cells. Suitable materials are given herein, but some preferred are based on polysiloxane based elastomers as for example mentioned hereinbefore. Such elastomers also have low autofluorescence which could interfere with known cell staining experiments. The thickness of the membrane may be tuned together with the optical properties of the membrane material to set a desired transmissivity.

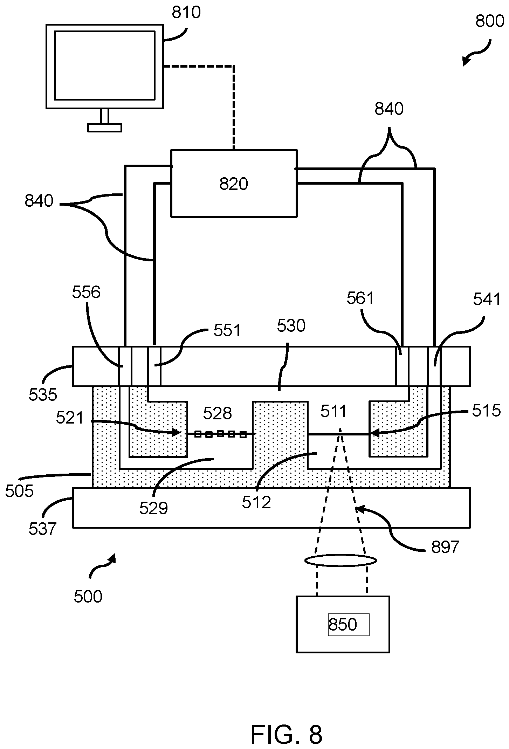

Fig. 8 conceptually depicts a testing system 800 according to an embodiment.

The cell culture testing system comprises a processing system 810, a flow control system 820 and a fluidic device 500. The fluidic device assembly 500 is illustrated in cross-sectional view. The fluidic device 500 is commonly carried in a device holder which is not shown for clarity. Any holder may be used allowing and providing the function needed as for the system and as described herein. For example, the holder may comprise a clamping mechanism capable of releasably clamping the substrate 505 between the cover plates 535 and 537 such that the fluidic device is leak free. The system is shown having the device 500, but such system may have any one of the devices described herein.

The flow control system 820 is fluidically coupled to the inlets 551 and 556 and to the outlets 541 and 561 through pipes or tubing 840.

The flow control system 820 is configured to control the flow of liquid or fluid through the fluid channels of the fluidic device 500. In particular, the flow control system may comprise one or more pumps for controlling the flow of media or fluid through each fluid channel of the fluidic device. Thus, the flow control system can control the rate of fluid flow and/or amount of fluid through the fluidic device 500 via the input and output fluid channel(s) as required by the cases of the fluidic devices.

Thus, a flow control system 920 may comprise one or more pumps for controlling the flow of liquid at least into the fluidic device. Examples are disclosed, for instance, by: Laser, Daniel J., and Juan G. Santiago. "A review of micropumps." Journal of micromechanics and microengineering 14.6 (2004): R35; Foret, Franta, and Jörg P. Kutter. "Pumps for microfluidic cell culture." (2014); or Narayanamurthy, Vigneswaran, et al. "Advances in passively driven microfluidics and lab-on-chip devices: A comprehensive literature review and patent analysis." RSC advances 10.20 (2020): 11652-11680.

The processing system 810 is configured to control the operation of the flow control system 820, e.g., the operation of any pumps housed by the flow control system. The control by the processing system 810 may be according to any desired emulation or simulation technique. The processing system may for example be embodied by a workstation or other computer running software programs for providing input to the flow control system. Such systems may be completely standard and known in the art.

The system 800 may further include an optical inspection device 850 for observing cell culture or cells within one or more of the chambers 511 and 528. In this case, and as most usual, the optical inspection device collects optical radiation 897 in the visual light range from the bottom. As such the beam must pass the perforated membrane 515 and/or the supporting member 521 in addition to part of the substrate 505 and the cover plate 537. Hence, all of these preferably are at least to some extent transparent to the optical radiation involved. Preferably the fluidic device 500 (or any other for that matter) has sub chambers 512 and/or 529 extending completely through the entire thickness of substrate 505 to be bound by the cover plate 537 (as for example indicated for chambers 210 of Figs. 2A and 2B). In that way the optical radiation does not need to pass the substrate 505 on its bottom.

While embodiments disclosed herein have been described with reference to use in investigation and/or treatment of cancerous cell cultures, and in particular of the metastasis behavior of cancerous cell cultures, other use scenarios as described herein can be thought of.

In summary, the current disclosure provides a fluidic device comprising a monolithic substrate. The substrate defines a first chamber, a second chamber and a fluid channel connecting the two chambers. The first chamber supports a perforated membrane that divides the first chamber into a first sub-chamber and a second sub-chamber. The second chamber is configured for supporting a cell culture. The substrate is configured such that fluid introduced to the second chamber flows to the first sub-chamber of the first chamber, before passing through the perforated membrane to the second sub-chamber. Thus, fluid introduced to the first sub-chamber via the fluid channel can only reach the second sub-chamber via the perforated membrane.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

According to additional examples in accordance with an aspect of the invention, there is provided a fluidic device comprising an integrally formed substrate. The integrally formed substrate comprises a first chamber supporting a perforated membrane that divides the first chamber into a first upper sub-chamber and a first lower sub-chamber, the perforated membrane comprising a plurality of pores having a diameter of no more than 9µm for capturing cells; a second chamber for culturing a cell culture; and a fluid channel that fluidly connects the second chamber to the first upper sub-chamber, to allow fluid introduced to the second chamber to transport cells from the second chamber to the first upper sub-chamber. The first chamber is configured such that fluid introduced to the first upper sub-chamber via the fluid channel passes from the first upper sub-chamber to the first lower sub-chamber only via the perforated membrane.

*The present disclosure provides a single, monolithic device for use in emulating or analyzing cell emission from a cell culture to a fluid motion. Such a device could, for instance, be used to test or analyze the effect of different treatments on cancerous cell culture, e.g., by analyzing the effect on the amount and*/*or condition of cells released by a cell culture. Thus, the proposed fluidic device can be used to emulate or simulate metastasis.*

*The substrate of the fluidic device is formed of a single piece of material, and comprises two chambers that are fluidly connected together. One of the chambers (the "first chamber") houses or supports a perforated membrane. The perforated membrane may be integrally formed with the substrate (e.g., be formed as part of the substrate) or be a separate element. Fluid flows from the other chamber (the "second chamber") to the first chamber, carrying or transporting (any) cells to the perforated membrane. The fluid then moves through the perforated membrane, e.g., under the action of gravity and*/*or fluid pressure. The cells carried by the fluid are captured by the pores of the perforated membrane due to their relatively small size. The captured cells can then be investigated, e.g., by microscope or the like, in order to analyze or assess their condition, number and*/*or shape.*

*In this way, it is possible to assess cells that are released or emitted by any cell cultures that are provided in the second chamber. The second chamber can therefore be used to perform a treatment on the cell culture, such as the application of a certain therapeutic fluid or drug (e.g., a chemotherapy treatment fluid), with the first chamber capturing any released cells to analyze the effect of the treatment performed in the second chamber on metastasis.*

Preferably, the width of the first chamber is no less than 0.5 mm; and the width of the second chamber is no less than 1 mm.

The second chamber may house a supporting member comprising a plurality of separate wells, each well configured for culturing a different cell culture.

The fluidic device may comprise an output fluid channel fluidly connected to the first lower sub-chamber to allow the output or extraction of fluid from the first lower sub-chamber. *This allows waste fluid to be siphoned away from the first chamber, e.g., allowing for fluid to continually flow through to the perforated membrane and avoiding spillage.*

In preferred examples, the first chamber is fluidly connected to the second chamber via only one or more channels formed in a single side of the fluidic device. *This technique provides a simple, materially efficient and easy to manufacture technique that provides many, if not all, of the required fluid channels for assessing the effect of metastasis.*

Preferably, only a single fluid channel connects the first chamber to the second chamber.

In some examples, the second chamber supports a second perforated membrane that subdivides the second chamber into a second upper sub-chamber and a second lower sub-chamber. The fluid channel may fluidly connect the first upper sub-chamber to the second upper-sub-chamber.

In at least one example, the second perforated membrane comprises: a supporting structure having a substantially planar surface; a plurality of wells formed in the supporting structure, each well having a width of between 200µm to 800µm and being configured to extend from the second upper sub-chamber into the second lower sub-chamber; and for each of the plurality of wells, one or more second pores positioned in the well, each second pore having a width of no more than 9µm.

Optionally, each pore of the perforated membrane has a width of between 5µm and 9µm.

In at least one example, the integrally formed substrate comprises an input fluid channel extending from the second chamber and only fluidly connected to the first chamber via the second chamber; and the input fluid channel comprises an exposed portion for receiving fluid for the second chamber.

Preferably, the integrally formed substrate comprises an upper planar surface and the first and second chambers are formed as recesses in the upper planar surface.

In some examples, the perforated membrane is formed from a rubber or rubber-derived material.

In some examples, the fluidic device has a visible transmittance of more than 0.8. *This increases an ease for a user of the fluidic device to monitor and*/*or assess any cells or particles captured by the perforated membrane or elsewhere in the fluidic device.*

There is also proposed a drug testing method comprising: obtaining the fluidic device herein described; providing a cell culture to the second chamber; exposing the cell culture to a drug to be tested in the second chamber; introducing a fluid to the second chamber such that the introduced fluid flows from the second chamber, through the fluid channel and through the perforated membrane supported by the first chamber; and assessing any cells captured by the perforated membrane.

## Claims

1. A fluidic device comprising an integrally formed substrate, the integrally formed substrate comprising:
a first chamber for housing a perforated membrane that divides the first chamber into a first sub-chamber and a second sub-chamber;
a second chamber including for culturing cells or tissue;
the fluidic device comprising:
the perforated membrane housed within the substrate;
a first fluid channel connecting the first sub-chamber to the second chamber such that fluid can flow from the second chamber to the first sub-chamber via the first fluid channel,
the perforated membrane comprising a plurality of pores having a diameter of 10 µm or less.

2. The fluidic device of claim 1, wherein one or more of the fluid channel and the perforated membrane is part of the integrally formed substrate.

3. The fluidic device of any of the previous claims further comprising a supporting member for supporting the cell culture, the supporting member arranged in the substrate to divide the second chamber into a third sub-chamber and a second sub-chamber such that the third sub-chamber is connected to the first sub-chamber via the first fluid channel.

4. The fluidic device of claim 3, wherein the perforate membrane is a first perforated membrane and the supporting member comprises a second perforated membrane comprising a plurality of second pores each having a diameter of 10 µm or less.

5. The fluidic device of claim 3 or 4, wherein the supporting member comprises a plurality of separate wells, each well for culturing a separate one of a plurality of cell cultures, each well having an opening facing the third sub-chamber.

6. The fluidic device of claim 5, wherein each well opening has a diameter in the range of 200 µm to 1000 µm.

7. The fluidic device of claim 3 or 4, wherein the supporting member comprises a slit for holding a biopsy.

8. The fluidic device of any of the previous claims, further comprising one or more further channels of the group of channels consisting of:
- a first input fluid channel 350 connected to the second chamber and a first inlet for introducing fluid to the second chamber ; and
- a first output fluid channel connected to the second sub-chamber and a first outlet for removing fluid from the second sub-chamber; and
- a second input fluid channel connected to the fourth sub-chamber and a second inlet for removing fluid from the fourth sub-chamber; and
- a second output fluid channel connected to the first sub-chamber and a second outlet for removing fluid from the first sub-chamber.

9. The fluidic device of claim 4, comprising a bridging channel connecting the second sub-chamber to the fourth sub-chamber such that fluid can flow between the second and fourth sub-chamber via the bridging channel.

10. The fluidic device of claim 4 or 9, comprising a bypass channel 392 connected to the fourth sub-chamber and a fluid outlet.

11. The fluidic device of any of the previous claims wherein the first sub-chamber, the second sub-chamber, the perforated membrane, the second chamber, the first fluid channel and, if present, the supporting member define a dual chamber unit, and the fluidic device comprises a plurality of the dual chamber units.

12. The fluidic device of claim 11 wherein the plurality of dual chamber units are arranged such that:
- their first sub-chambers are connected in parallel to a joint second fluid output channel connected to a second outlet;
- their second sub-chambers are connected in series to a joint first output fluid channel between a further inlet and a first outlet;

13. The fluidic device of claim 11 or 12, wherein the plurality of dual chamber units are arranged such that:
- their third sub-chambers are connected in parallel to a joint first input fluid channel connected to a first inlet; and,
- optionally, when the fluidic device comprises a supporting member as defined in claim 4, their fourth sub-chambers are connected in series to a joint second input fluid channel between a second inlet and a further outlet.

14. The fluidic device of any of claims 1 to 12, wherein the perforated membrane comprises an elastomeric material, preferably a polysiloxane based elastomeric material, more preferably a polysiloxane based elastomeric material comprising carboxylic acid groups.

15. The fluidic device of any of claims 1 to 13 having a visible light transmittance of more than 0.8.

16. A drug testing method comprising:
providing a cell culture to one or more second chambers of a fluid device as claimed in claims 1 to 14;
exposing the cell culture to a drug in the second chamber;
causing a fluid flow from the second chamber to the first sub-chamber suitable to transport cells emitted by the cell culture, if any, to the first sub-chamber; and
assess whether any emitted cells have been retained in the first sub-chamber.
